(19) Europäisches Patentamt
European Patent Office
Office européen des brevets

(11) Publication number: **0 345 827 A1**

# (12) EUROPEAN PATENT APPLICATION

(21) Application number: **89113483.5**

(51) Int. Cl.[4]: **C07D 499/00 , A61K 31/43**

(22) Date of filing: **21.11.83**

(30) Priority: **29.11.82 US 445295**
**17.01.83 US 458511**
**28.01.83 US 461845**
**31.01.83 US 462723**

(43) Date of publication of application:
**13.12.89 Bulletin 89/50**

(60) Publication number of the earlier application in accordance with Art.76 EPC: **0 110 280**

(84) Designated Contracting States:
**AT BE CH DE FR GB IT LI LU NL SE**

(71) Applicant: **SCHERING CORPORATION**
**2000 Galloping Hill Road**
**Kenilworth New Jersey 07033(US)**

(72) Inventor: **Girijavallabhan, Viyyoor Moopil**
**10 Maplewood Drive**
**Parsippany New Jersey 07054(US)**
Inventor: **Ganguly, Ashit Kumar**
**96 Cooper Avenue**
**Upper Montclair New Jersey 07043(US)**
Inventor: **Pinto, Patrick Anthony**
**232 Randolph Drive**
**Mine Hill New Jersey 07801(US)**
Inventor: **Versace, Richard William**
**13 Edgewood Road**
**Ringwood New Jersey 07456(US)**
Inventor: **McCombie, Stuart Walter**
**159 Pleasant Valley Way**
**West Orange New Jersey 07052(US)**

(74) Representative: **Ritter, Stephen David et al**
**Mathys & Squire 10 Fleet Street**
**London EC4Y 1AY(GB)**

## (54) Process for the production of penem compounds.

(57) Compounds of the general formula XII having valuable antibacterial activity

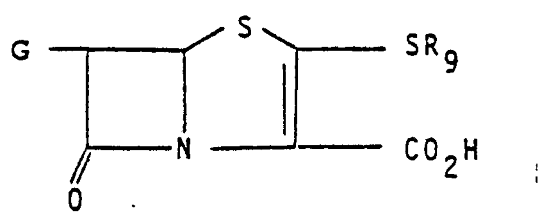

XII

are provided in which G is 1-hydroxyethyl, Rg is chosen from the group

EP 0 345 827 A1

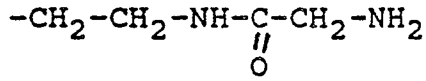

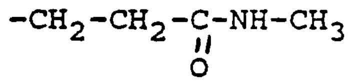

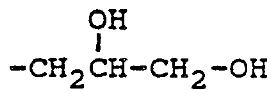

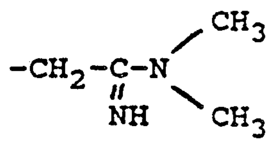

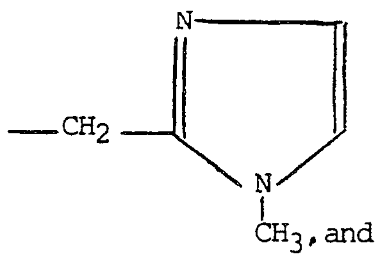
, and

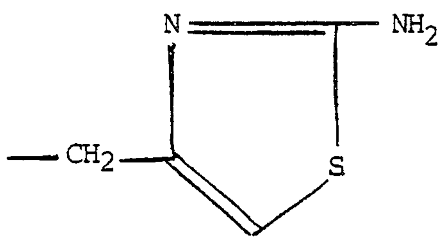

# PROCESS FOR THE PRODUCTION OF PENEM COMPOUNDS

This invention relates to processes for preparing 6-hydroxyalkyl-2-substituted thio-penem-3-carboxylic acids, and their salts and esters (referred to herein as penems) to certain novel penems and pharmaceutical compositions containing them.

Penems, a recent addition to the family of synthetic β-lactams, possess potent anti-bacterial activity. They have, hitherto been prepared by laborious, time consuming, multistep processes which result in low yields and are thus uneconomical.

According to one aspect of the present invention we provide a process for the preparation of penem compounds of formula I

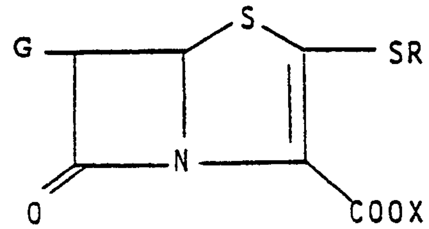

I

wherein R is hydrogen or an organic radical, and G is hydroxy-loweralkyl, X is hydrogen, a pharmaceutically acceptable salt forming group e.g. an alkali metal cation, a pharmaceutically acceptable ester group or a carboxy protecting group,
and the tautomers thereof when R is hydrogen, characterised in that,

(a) a compound of formula II

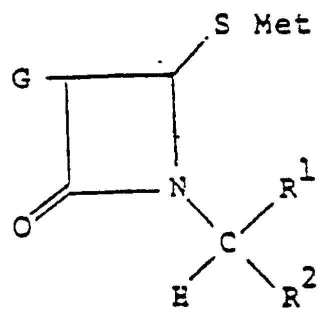

II

wherein $R_1$ and $R_2$ are protected carboxy groups which may be the same or different and Met is a thiophilic metal atom, is reacted with a compound of formula III

$S=C(-Y)_2$ III

in which Y is a leaving group, to produce a compound of formula IV

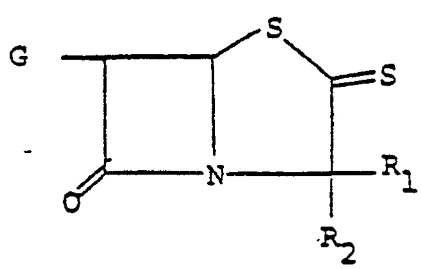

IV

followed by removal of the protected carboxy group $R_1$ whereby a tautomeric compound wherein R is hydrogen (i.e. a compound of formula Va in equilibrium with its tautomer Vb)

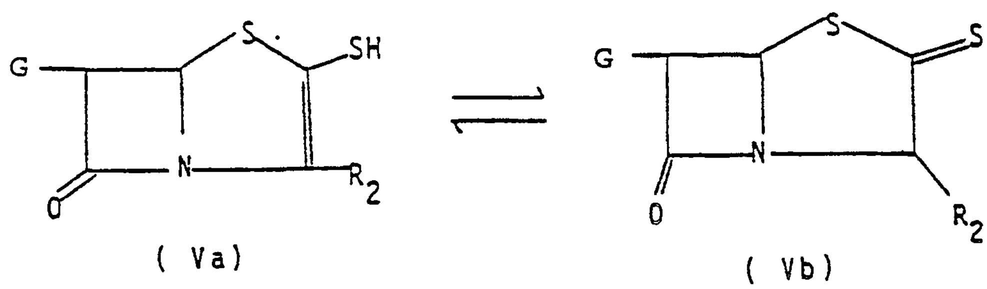

( Va ) ( Vb )

in which $R_2$ is as defined above is obtained,
and that, if desired such tautomeric compound is transformed into a compound of formula I wherein R represents an organic radical by introducing such R by known conventional methods;

(b) a compound of formula VI

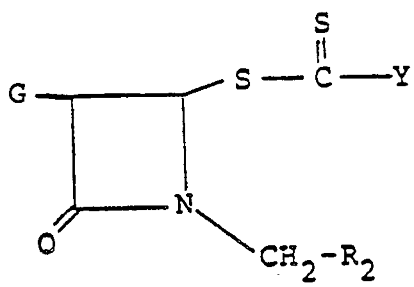

VI

wherein $R_2$ and Y are as defined above, is subjected to intramolecular cyclisation whereby a tautomeric compound [(V a), (V b)] as defined in process (a) above is obtained, followed, if desired, by transformation into a compound of formula I wherein R represents an organic radical, as under process (a) above;

(c) for the preparation of a compound of formula I wherein R represents

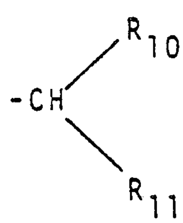

wherein either $R_{10}$ is trifluoroloweralkyl and $R_{11}$ is selected from trifluoroloweralkyl and dihydroxy loweralkyl; or
$R_{10}$ is hydrogen and $R_{11}$ is chosen from 2-amino-4-thiazolyl, 5-amino-2-thiazolyl, 5-nitro-2-thiazolyl, 1-methyl-2-imidazolyl, aminoacetylaminomethyl, N-methylcarbamoylmethyl, dihydroxyloweralkyl,

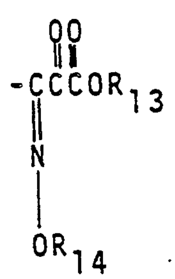

and

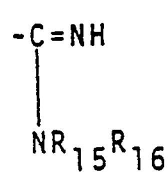

whereby in the above groups
$R_{13}$ and $R_{14}$ are independently hydrogen or loweralkyl, and

$R_{15}$ and $R_{16}$ are independently hydrogen or loweralkyl or
$R_{15}$ and $R_{16}$ together with the nitrogen to which they are attached form a heterocyclic ring having 3 to 6 ring members,
a tautomeric compound of formula [(V a), (V b)] as defined under process (a) above is subjected to an alkylation reaction introducing the group $-CHR_{10}R_{11}$;

(d) for the preparation of compounds of formula I wherein R is as defined in process (c) above, reacting a compound of formula VII

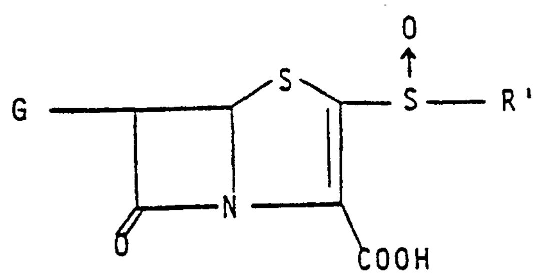

VII

in which R′ is an organic group different from the desired group R with a thiol of formula VIII′ or reactive derivate thereof
R—SH VIII′
in which R is as defined in process (c);

(e) for the production of a compound of formula I in which R is as defined in process (c) above, reacting a compound of formula IX

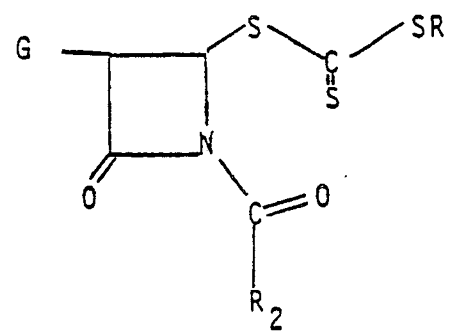

IX

with a trivalent organophosphorous compound, wherein in the processes (a) to (e) above, any functional groups in the reactants may be protected by protecting groups, which are removed at any appropriate stage of the process, and a so-obtained compound of formula I, if desired, is subjected to deprotection of a protected carboxyl group $R_2$ (if present) and is isolated as the free acid, as a pharmaceutically acceptable salt or pharmaceutically acceptable ester.

Among the methods used in processes (a), (b) and (c) for transforming a tautomeric compound of formula [( Va), ( Vb)] into another compound of formula I it is preferred to use one or other of the following:

(i) reaction with a compound of the formula
RZ
in which R is an organic group, as defined in processes (a), (b) and (c) and Z is a leaving group. Typical leaving groups Z are halides, e.g. chlorine, bromine and iodine, hydroxyl and trichloromethylsulfonyl;

(ii) olefin addition, for example using a 1,2-unsaturated unsubstituted or substituted $C_2$-$C_6$ alkylene whereby a compound of formula I in which R is an unsubstituted or substituted $C_2$-$C_6$ alkyl group is produced;
This type of reaction is useful for example in the production of 2-alkylthio substituted penems. By using olefines of the formula
$R_3CH=CH_2$
in which $R_3$ is hydrogen or lower alkyl e.g. methyl, ethyl or propyl, compounds of formula I in which R is ethyl, propyl, butyl and pentyl can be obtained;

(iii) reaction with a substituted or unsubstituted 1,2-epoxy $C_2$-$C_6$ alkane, whereby a compound of formula I in which R is a $C_2$-$C_6$ alkyl group having a hydroxy group attached to the second carbon atom from the sulphur atom and optionally carrying one or more other substituents is obtained.
This type of reaction can for example, thus be used for producing compounds of formula I in which R is e.g.

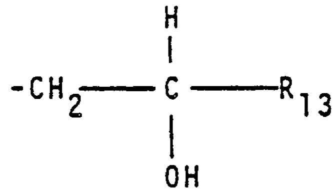

in which $R_{13}$ is hydroxyloweralkyl or carboxyloweralkyl in which the carboxy group may be esterified or salified. By using glycidol a compound of formula I in which R is 2,3-dihydroxy-propyl can be obtained for example; glycidic acid and alkali metal glycidates lead to compounds of formula I in which R is 2-hydroxy-3-carboxyl propyl and alkali metal salts thereof respectively,

According to a further aspect of the present invention are novel compounds of the formula XIII

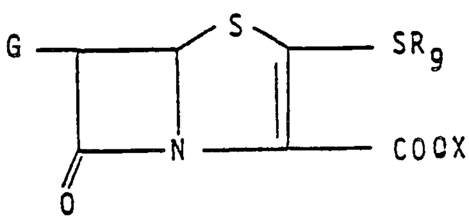

XIII

in which $R_9$ is chosen from the group

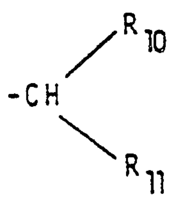

in which $R_{10}$ is trifluoroalkyl and $R_{11}$ is trifluoroalkyl or dihydroxyloweralkyl; or
$R_{10}$ is hydrogen and $R_{11}$ is chosen from 2-amino-4-thiazolyl, 5-amino-2-thiazolyl, 5-nitro-2-thiazolyl, 1-methyl-2-imidazolyl, aminoacetylaminomethyl, N-methylcarbamoylmethyl, dihydroxyloweralkyl,

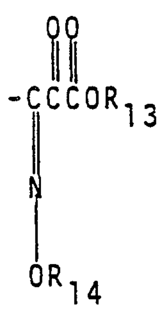

and

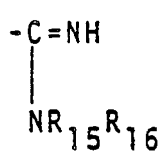

wherein in the above groups $R_{13}$ and $R_{14}$ are independently hydrogen or loweralkyl, and
$R_{15}$ and $R_{16}$ are independently hydrogen or loweralkyl or
$R_{15}$ and $R_{16}$ together with the nitrogen to which they are attached form a heterocyclic ring having 3 to 6 ring members, and the pharmaceutically acceptable salts or metabolisable esters thereof.

Group G may for example be hydroxymethyl, 1-hydroxyethyl or 2-hydroxyprop-2-yl. Preferably G is 1-hydroxyethyl; and for convenience the invention is illustrated in the following description with reference to compounds in which G is 1-hydroxyethyl, it being understood that other hydroxyalkyl groups can be used instead of said 1-hydroxyethyl group.

For convenience the tautomeric compound [(Va), (Vb)] will be referred to below simply as the compound V.

Processes (a) and (b) defined above constitute novel and inventive processes capable of producing known and novel penems in high yield.

Penems of formula XIII are novel and inventive compounds produceable by the novel processes (a) and (b) and also by processes (c), (d) and (e) defined above.

Certain compounds of formula I are known to be useful as antibiotics or as valuable intermediaries in the preparation of penem antibiotics.

Compounds of formula XIII are useful as antibiotics, being active against both gram positive and gram negative organisms such as Staphylococcus aureus, Escherichia coli and Pseudomnas aeruginosa.

For instance compounds of the formula XIII when tested in standardized microbiological assays, are found to be active against such gram-positive organisms as Staphylococcus epidermis and Bacillus subtilis and such gram-negative organisms as E. coli and Salmonella at test levels of 0.1 to 2.0 micrograms/ml. Additionally they show activity against organisms which produce beta-lactamases, e.g. penicillinase and cephalosporinase, indicating a resistance against these enzymes.

Thus a further aspect of the present invention comprises a pharmaceutical composition comprising an anti-bacterially effective amount of a penem of formula XIII together with a compatible, pharmaceutically acceptable carrier or excipient.

The dosage administered of the novel penems of this invention is dependent upon the age and weight of the animal species being treated, the mode of administration, and the type and severity of bacterial infection being prevented or reduced. Typically, the dosage administered will be in the range of 1 to 250 mg/kg, with 5 to 20 mg/kg in divided dosages being preferred.

The novel penems of this invention may be administered either orally or parenterally. Preferably, the compounds are administered orally.

For oral administration, the antibacterial compounds of this invention may be formulated in the form of tablets, capsules, elixirs or the like. Likewise, they may be admixed with animal feed. They may also be applied topically in the form of ointments, both hydrophilic and hydrophobic, in the form of lotions which may be aqueous, non-aqueous or of the emulsion type, in the form of creams, or by mechanical delivery systems, e.g. transdermal.

The compounds of formula XIII may also be utilized in liquid form such as solutions, suspensions and the like for otic and optic use and may also be administered parentarally via intramuscular injection.

As used herein, "pharmaceutically acceptable salts" means alkali metal salts such as sodium and potassium salts; alkaline earth metal salts such as calcium, magnesium and aluminum salts; amine salts formed from suitable organic amines, i.e., aliphatic, cycloaliphatic, (cycloaliphatic)aliphatic or araliphatic primary, secondary or tertiary mono-, di- or polyamines, or heterocyclic bases, e.g., salts derived from triethylamine, 2-hydroxyethylamine, di-(2-hydroxyethyl)amine, tri-(2-hydroxyethyl)amine, 4-aminobenzoic acid-2-diethylaminoethyl ester, 1-ethylpiperidine, bicyclohexylamine, N,N′-dibenzylethylenediamine, pyridine, collidine, quinoline, procaine, dibenzylamine, 1-ephenamine and N-alkylpiperidine. Acid addition salts formed from mineral acids such as hydrochloric, hydrobromic, hydroiodic, phosphoric or sulfuric acids, or formed from organic carboxylic or sulfonic acids such as trifluoroacetic, para-toluene sulfonic, maleic, acetic, citric, oxalic, succinic, benzoic, tartartic, fumaric, mandelic, ascorbic and malic acids.

Suitable pharmaceutically acceptable ester groups are those known in the penicillin, cephalosporin and penem arts to be cleavable from the parent compound, within the body, to give the corresponding acid.

Example of such esters are indanyl,phthalidyl, methoxymethyl, glycycloxymethyl, phenylglycyloxymethyl, thienylglycyloxymethyl, acetoxymethyl and pivaloyloxymethyl.

Suitable carboxyl protecting groups are those which can be removed under conventional conditions without reacting with other functional groups present on the β-lactam, for example allylic, cyanoloweralkyl, loweralkylsilylloweralkyl, sulfone esters, p-nitrobenzyl and trichloroethyl. For instance the preferred protecting group in $R_2$ is allyl; and in $R_1$ and $R_2$, in compound II, the preferred protecting groups are preferably respectively loweralkylsilylloweralkyl and allyl.

Unless otherwise stated, the term "loweralkyl" includes branched- and straight-chain alkyl groups of from 1 to 6, preferably 1 to 4, carbon atoms and includes, for example, methyl, ethyl, n-propyl, isopropyl, t-butyl, pentyl and hexyl.

The term "organic radical" includes the groups lower alkyl; loweralkyl substituted with one or more

substituents independently selected from halogen, hydroxy, lower alkoxy, cyano, oxo, carb(lower)alkoxy, carbamoyl, N-lower-alkyl-substituted-carbamoyl, N,N-diloweralkyl substituted-carbamoyl, amino, loweralkylamino, diloweralkylamino, lower alkanoylamino, aminoacylamino, alkylthio, arylthio, heterocyclic, heterocyclicthio,

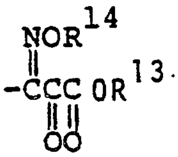 ; 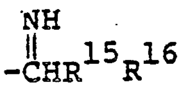

(wherein $R^{14}$ and $R^{13}$ are independently hydrogen or lower alkyl and $R^{15}$ and $R^{16}$ are each lower alkyl or $R^{15}$ and $R^{16}$ together with the nitrogen to which they are attached form a heterocyclic ring of 3 to 6 members) butanolidyl, aryl, heteroaryl or aryl or heteroaryl substituted with 1 to 4 substituents independently selected from loweralkyl, hydroxy, lower-alkoxy, halogen, haloloweralkyl, loweralkylthio, amino, loweralkylamino, diloweralkylamino, carboxyloweralkyl, nitro, and cyano;
aryl or aryl substituted with 1 to 4 substituents e.g. independently selected from loweralkyl, hydroxy, lower alkoxy, halogen, haloloweralkyl, loweralkylthio, amino, loweralkylamino, diloweralkylamino, carboxyloweralkyl, nitro, cyano, aminoalkyl and carboxy; and
heteroaryl or heteroaryl substituted with 1 to 4 substituents independently selected from loweralkyl, hydroxy, lower alkoxy, halogen, haloloweralkyl, lower alkylthio, loweralkylsulfonyl, amino, loweralkylamino, diloweralkylamino, carboxyloweralkyl, nitro and cyano.

The term "halogen" means fluoro, chloro and bromo.

The term "loweralkanoylamino" means the group

$-N\overset{\overset{O}{\|}}{C}R^{17}$ wherein $R^{17}$ is lower alkyl.

The term "aryl" means aromatic groups of 6 to 10 carbons and includes phenyl, naphthyl and the like. For example, "aryl" may be phenyl or phenyl substituted by one or more substituent groups for example, chloro, bromo, fluoro, loweralkyl, hydroxy, nitro, amino, aminomethyl, lowermonoalkylamino, lowerdialkylamino, loweralkoxy and carboxy. Such substituted aryl groups especially in $R'_2$ (see below) can be, for example, 4-hydroxyphenyl, 3,4-dichlorophenyl, 2,6-dimethoxyphenyl, 4-methylphenyl, 2-fluorophenyl, 4-carboxyphenyl, 3-nitrophenyl, 4-aminophenyl, 3-aminophenyl, 4-dimethylaminophenyl, 4-aminomethylphenyl and 4-ethoxyphenyl.

The term "heterocyclic" preferably means cyclic groups of 5 or 6 ring atoms wherein 1 to 4 ring atoms are selected from nitrogen, sulfur and oxygen and includes for instance piperidyl, piperizinyl, pyrrolidinyl, thiazolidinyl, thiomorpholinyl, morpholinyl, tetrahydrofuranyl, tetrahydropyranyl, tetrahydrothiofuranyl, and tetrahydrothiopyranyl. Also included are the positional isomers of the above, e.g. 3-piperidinyl and 4-piperidinyl, 2-pyrrolidinyl and 3-pyrrolidinyl.

The term "heteroaryl" preferably means aromatic heterocyclic groups of 5 to 10 ring atoms wherein 1 to 3 ring atoms are selected from the group consisting of nitrogen, sulfur and oxygen and includes for instance imidazolyl, pyridinyl, pyrimidinyl, thiazolyl, pyrrolyl, pyrazolyl, pyrazinyl, furyl, thiofuryl, triazolyl, oxazolyl, 1,2,3-oxadiazole, indolyl, benzothiofuranyl, tetrazolyl and the like.

The term "removable hydroxy protecting group" means any such group conventionally used for this purpose, with the only requirement being compatability with the hydroxy substituent on the penems and β-lactam intermediates and removability utilizing elemental zinc or any other conventional agent for this purpose which will not adversely affect the penem or β-lactam intermediates structure. The preferred hydroxy protecting groups include trichloroethoxycarbonyl, dimethyltri butylsilyl, trimethylsilyloxycarbonyl and trimethylsilyl.

The process of this invention can be used to produce penems of any desired stereochemistry. The most preferred stereochemistry of the final penem compounds is (5R,6S,8R), less preferred is (5R,6R,8S). Final penems of desired stereochemistry can be obtained by selection of starting material of appropriate stereochemistry.

In process (a): the conversion of metal salt II into thione IV is typically conducted, in a suitable solvent. When metal salt II is obtained according to reactive scheme 1, step B, as described below it may be used directly, i.e. without isolation from the reaction mixture in which it is formed. Thus the same solvent may be used in this step as in the preceding step.

Temperature will usually be in the range from about 10 °C to about 45 °C; about 25 °C is preferred. Leaving group Y in the thiocarbonyl reagent III is typically chloro, bromo, iodo, or imidazolyl. 1,1-thiocarbonyldimidazole is preferred as reagent III because of its crystalline nature and ease of use.

The removal of one of the protected carboxy protecting groups at position 3 of the thione IV to afford the tautomer [( Va), ( Vb)] is typically conducted in a suitable organic solvent such as tetrahydrofuran, ethylether or dioxane at a temperature in the range of from about 10 °C to about 45 °C, about 25 °C being preferred. The removal is usually effected by the addition of one functional equivalent of fluoride ion so that only a single protected carboxy group is removed.

In one embodiment of the invention $R_2$ is allylicoxycarbonyl, preferably

$-\overset{O}{\overset{\|}{C}}-OCH_2CH=CH_2$ and $R_1$ is

$-\overset{O}{\overset{\|}{C}}-OCH_2CH_2R_1'$ wherein $R_1'$ is a loweralkylsilyl group, especially trimethylsilyl, t-butyldiphenylsilyl or an equivalently functioning group. In such cases, particularly when $R_2$ is

$-\overset{O}{\overset{\|}{C}}-OCH_2CH=CH_2$ and $R_1$ is trimethylsilylethoxycarbonyl it will be ensured that it is $R_1$ which is removed by treatment with fluoride ion.

Typically, tetrabutylammoniumfluoride (TBAF) is used as the fluoride ion source, although any other suitable source of fluoride ion may similarly be used to provide one functional equivalent of fluoride ion, e.g. $C_sF$ or KF.

For instance a stoichiometric excess of the fluoride ion source may be used provided this gives rise to the presence of only one functional equivalent in the reaction mixture. This is true particularly for TBAF. Moreover, because TBAF dissociates slowly in these solutions and as removal of the allyl protecting group (preferred for $R_2$) is much slower than removal of the trimethylsilyl protected carboxy group (preferred for $R_1$), an excess (e.g. 2 equivalents) of TBAF results in only one functional equivalent of fluoride ion being employed in this reaction step.

The tautomeric compound of formula V may be isolated at this stage for synthesis of other penems, as described in more detail below.

In process (b):

the cyclization of the compound of formula VI into the tautomeric compound V is typically carried out in an anhydrous inert organic solvent, for example, tetrahydrofuran, and a non-neuclophilic strong base, for example, lithium diisopropyl amide (LDA) and lithium di-(trimethylsilyl)amine added to the system to effect cyclization. Usually cyclization will be carried out at from about -50 °C to -100 °C and preferably at about-70 °C and will generally be complete within about 5 minutes to 24 hours. Usually an essentially equimolar amount of the strong base will be used.

The transformation of a tautomeric compound V into another compound of formula I (in any of processes (a), (b) or (c) ) is typically carried out in an anhydrous inert organic solvent for example, tetrahydrofuran, ethylether or dioxane at temperatures ranging from about -10 °C to about 45 °C e.g. 10 °C to 45 °C with room temperature (about 25 °C) being preferred.

In the case of this transformation being carried out on a tautomeric compound V obtained via process (a) or (b) it will be noted that deprotection of the 8-position hydroxy group may previously be conducted, using suitable reagents, as discussed below, in such solvents and in a similar temperature range. Thus the transformation may be conducted as a continuation of such preceding deprotection step namely without isolation of the tautomeric compound V. Thus the same solvents will be used in both steps and it will also be usual to carry out reactions at about the same temperature, or at least in the same above mentioned temperature range in both steps. If the tautomer is, first, isolated, different solvent and temperature can be used in this step compared with the preceding step but, nevertheless, these will preferably be the same.

Transformation using a compound RZ is generally carried out in the presence of a base or acid acceptor. Such bases and acid acceptors known in the art for this reaction can be used e.g. inorganic bases such as alkali metal carbonates or bicarbonates, or organic bases such as triethylamine. Where tetrabutylammonium fluoride has previously been used (to remove $R_1$ in process (a) ) and remains in the reaction solution this also will function as a base in this reaction.

The alkylation step can, for example, be carried out, in one embodiment, by the reaction of a compound X of formula

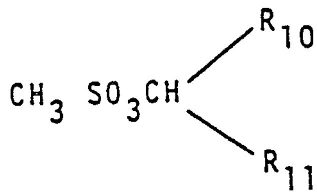

X

in which $R_{10}$ and $R_{11}$ are as defined above, with the tautomeric compound of formula V. This particular reaction will typically be carried out in a suitable organic solvent, for example, tetrahydrofuran. An essentially equimolar amount of an acid acceptor, for example an inorganic carbonate, facilitates the reaction. Typical reaction temperatures are in the range from about -5° C to about 30° C, and the reaction is generally complete within 1 to 24 hours.

Olefin addition is generally carried out using a radical initiator for example ABN[2,2′-azobis(2-methylpropionitrile)].

The removal of the protecting group from the protected carboxyl group $R_2$ can be carried out by conventional procedures selected according to the identity of the protecting group. The preferred protecting group is allylic, preferably allyl, and its removal can, in general be effected under catalytic conditions in the presence of a base, preferably by utilizing procedures described in our European Patent Application Publication No. 0013663. Thus the allylic groups are preferably removed by utilizing a suitable aprotic solvent, such as tetrahydrofuran, diethyl ether or methylene chloride, with an alkali metal alkylcarboxylate, preferably potassium or sodium 2-ethylhexanoate (to give the alkali meta penem salt, preferably the sodium or potassium penem salt, directly) or carboxylic acid, preferably 2-ethylhexanoic acid (to give the penem free-acid) and a mixture of a palladium compound and triphenyl phosphine as a catalyst. Most preferably this step procedes with the removal of the allylic protecting group and the formation of the alkali metal salt of the penem in situ.

If the product is a zwitterion deprotection of the allylic group requires only the catalyst and any mild neucleophile (e.g. $H_2O$ or alcohol).

In another, though less preferred embodiment protected carboxy groups $R_1$ and $R_2$ are both of the structure

$-\overset{O}{\overset{\|}{C}}-OCH_2CH_2R''_2$ in which both $R''_2$ s may be the same or different and chosen from cyano, loweralkylsilyl, e.g. trimethylsilyl or t-butyldiphenylsilyl, or sulfone ester of the formula $-SO_2R_x$ (wherein $R_x$ is an aryl group). or an equivalently functioning (electron withdrawing) group. Preferably both $R''_2$ groups are chosen from trimethylsilyl, t-butyldiphenylsilyl with the former most preferred.

When carrying out the above procedure in which both $R_1$ and $R_2$ have the structure

$-\overset{O}{\overset{\|}{C}}-OCH_2CH_2R''_2$ the removal of one carboxy group from the position 3 in the tautomeric compound of formula V is conveniently effected according to the procedure described above which uses one functional equivalent of fluoride ion preferably TBAF. Subsequent deprotection of the remaining carboxy protecting group $R_2$ can then be carried out (after the transformation reaction) by using reaction conditions and reagents identical to those used in the previous removal of the protected carboxy group. Preferably, the deprotection is carried out using tetrahydrofuran as solvent, at about 25° C with TBAF as the fluoride ion source.

This procedure will usually yield the free acid of the penem product. The formation of alkali metal salts and metabolisable esters can be carried out by treating the free acid according to the conventional procedures. For example, the free acid can be reacted with a stoichiometric amount of a suitable non-toxic base in an inert solvent followed by recovery of the desired salt by lyophilization or precipitation. Or a metabolisable ester can be formed by reaction of an alkali metal salt of a penem with a reactive derivate of the desired ester function; for example, the phthalidyl or pivaloyloxymethyl esters are preparable by reaction of the corresponding alkali metal penem salts with chlorophthalide or pivaloyloxymethylchloride in a solvent such as dimethylformamide, preferably with the addition of a catalytic amount of sodium iodide.

The intermediates of formula II are preparable, for example, by the reaction scheme I shown in the accompanying flow chart and in the procedures of this reaction scheme, as described below, it will be understood that any functional groups in any of the intermediates will be protected if necessary, or desired.

REACTION SCHEME 1

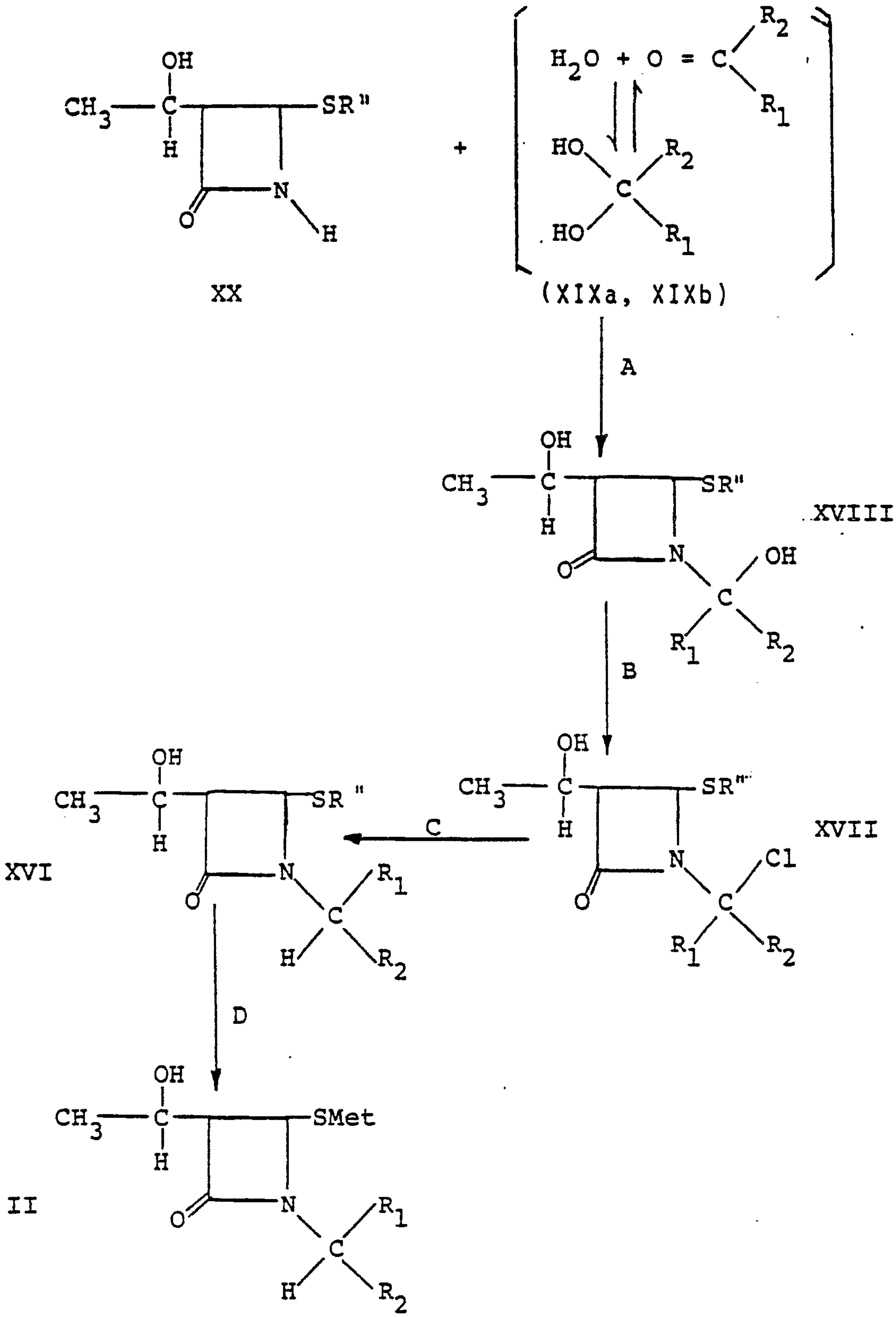


XX

(XIXa, XIXb)

XVIII

XVII

XVI

II

In the above reaction scheme intermediate XVIII is obtained in step A by reacting an azetidinone XX in which R″ is a sulphur protecting group, with the mixed ester (XIXa, XIXb), in which $R_1$ and $R_2$ are as defined above.

In this step A the reaction is typically conducted in a suitable organic solvent at about room temperature. Preferably the solvent is polar organic such as dimethylformamide; other solvents are for example tetrahydrofuran, acetonitrile and dimethylsulfoxide.

The mixed ester preferably has the formula

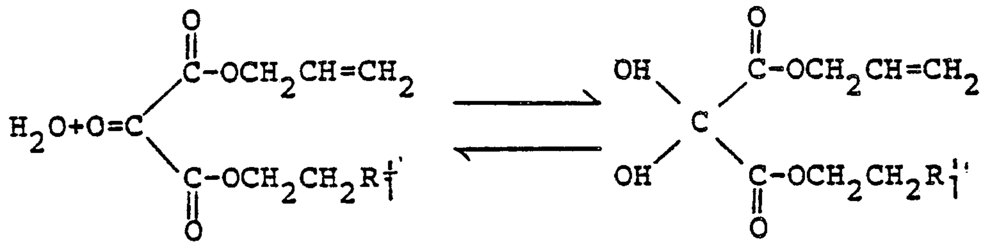

in which $R_1'$ is preferably chosen from trimethylsilyl, t-butyldiphenylsilyl or other equivalently functioning loweralkylsilyl groups. Preferred are trimethylsilyl and t-butyldiphenylsilyl groups with the former most preferred for reason of ready availability and ease of use.

Thus, referring to the above formulae, $R_1$ and $R_2$ preferably have the identities $-\overset{O}{\overset{\|}{C}}-OCH_2CH_2R_1'$

and $-\overset{O}{\overset{\|}{C}}-OCH_2CH_2=CH_2$

respectively.

The sulphur protecting group $R_1'$ in the azetidinone XX is preferably triphenylmethyl, 2-pyranyl or a loweralkylcarbonyl.

Step B. the chlorination of compound XVIII, is typically conducted in a suitable organic solvent (which may be the same solvent as that used in the preceding step) at temperatures of about -15° C to about 10° C in the presence of an acid acceptor. If the solvent is itself, or comprises, an acid acceptor, e.g. pyridine no additional acid acceptor is necessary. Alternatively, an organic solvent which is not an acid acceptor, for example, methylene chloride, chloroform, dimethyl formamide or acetonitrile may be utilized, and in such case a separate acid acceptor, organic or inorganic will be added to the reaction mixture. Typical of the suitable acid acceptors are organic bases, for example, pyridine or triethylamine and inorganic bases, for example, sodium or potassium carbonate. The chlorinating agent may be one conventionally used for the conversion of alcohols to chlorides for example, thionyl chloride, oxalyl chloride, phosphorous pentachloride, phosphorous trichloride or phosphorous oxychloride. Thionyl chloride is the most preferred.

The chlorination reaction may be carried out directly on the product of step A without isolation (viz the solvent can be the same as in the preceding step).

Step C, the dechlorination of compound XVII, can be carried out in any suitable organic solvent, for example, tetrahydrofuran, methylene chloride or dimethylformamide, and it may be carried out in the same solvent as is used for the preceding step, thus it may be carried out directly on the product of Step B, without isolation.

Water, or any proton source, adjusted by the addition of a mild acid, can be included to enhance the activity of the zinc. Typical reaction temperatures are in the range -15° C to about 25° C : 0° C is preferred.

In this step a protected 5-hydroxy group can be deprotected concomitantly with dechlorination if, as is preferred, the protecting group is one which is removable by elemental zinc.

If a hydroxy protecting group is present (at the 5 position) which is not so removable (by zinc) a separate removal step is conducted to remove it. Such removal steps are well known in the β-lactam art. This removal step, if required, can be carried out at any time after step C.

Step D can be carried out in a polar solvent, for example, methanol, ethanol, dimethylformamide, tetrahydrofuran or water. Suitable reactive salts of thiopholic metals are, for example, any reactive salts of copper, mercury, silver, lead, nickel and thallium in which the anion does not interfere in the reaction. It is preferred to use Cu(II), silver (I) and Hg(II), with silver (I) as most preferred. The salts may be salts or organic or inorganic acids, and suitable salts are, for example, silver nitrate, silver fluoborate, and silver acetate. Silver nitrate is the preferred salt. Typical copper salts are copper(II) acetate, and copper(II) nitrate.

A typical suitable mercury salt is mercuric acetate. In the case of lead salts it is observed that these give much slower reaction rates.

Silver salts are most preferred due to their ease of recovery and relative non-toxocity.

The reaction is facilitated by using an acid acceptor, e.g. pyridine or triethylamine. Preferably the reaction is carried out under an inert, e.g. nitrogen, atmosphere.

It is highly preferred, when carrying out the above procedures, that the β-lactam intermediates produced in each step are not isolated but remain in the reaction vessel and are treated according to the next reaction step. This facilitates the process to a very great extent as several steps can be carried out in the same solvent without regard to the separation of the desired product.

For instance, in a preferred embodiment of the process including the procedures of reaction scheme 1 the ester of formula (XIXa, XIXb) is added to the azetidinone of formula XX to form the intermediate of formula XVIII. This intermediate is then directly treated with the chlorinating agent, preferably thionyl chloride to form the chloride intermediate of formula XVII. This intermediate, again without isolation, is treated directly with elemental zinc to remove the chlorine atom and possibly concomitantly, if present, a hydroxy protecting group on the 5-substituent so as to afford the intermediate of formula XVI.

Thus the first steps A, B, C, and subsequent deprotection of the 5 position hydroxy group are conductable in the same reaction vessel, in the same solvent and this enables wastage caused by isolation of intermediate compounds to be mitigated.

In a highly preferred embodiment a compound of formula II in which the 5 position hydroxy group is not protected may be treated directly, without isolation from the reaction mixture in which it is formed, to protect the said hydroxy group; then without isolation the protected product may be subjected to the cyclization reaction with the thiocarbonyl compound of formula III, and thereafter, without isolation, the tautomeric product is treated to remove the 8 position hydroxy protecting group. Alternatively the aforesaid hydroxy protection and deprotection can be dispensed with and the compound of formula II used directly, without isolation, in the cyclization reaction with thiocarbonyl of formula III.

It is likewise preferred to dispense with the isolation of the tautomeric product (V a, V b) when working up or preparing other compounds of formula I; thus the various transformation procedures can be carried out directly without separation of the tautomer from the reaction mixture in which it was prepared, and the resulting compound of formula I can be subjected to removal of the carboxy protecting group in $R_2$, likewise, directly without isolation from the reaction mixture in which it was formed.

## REACTION SCHEME 2

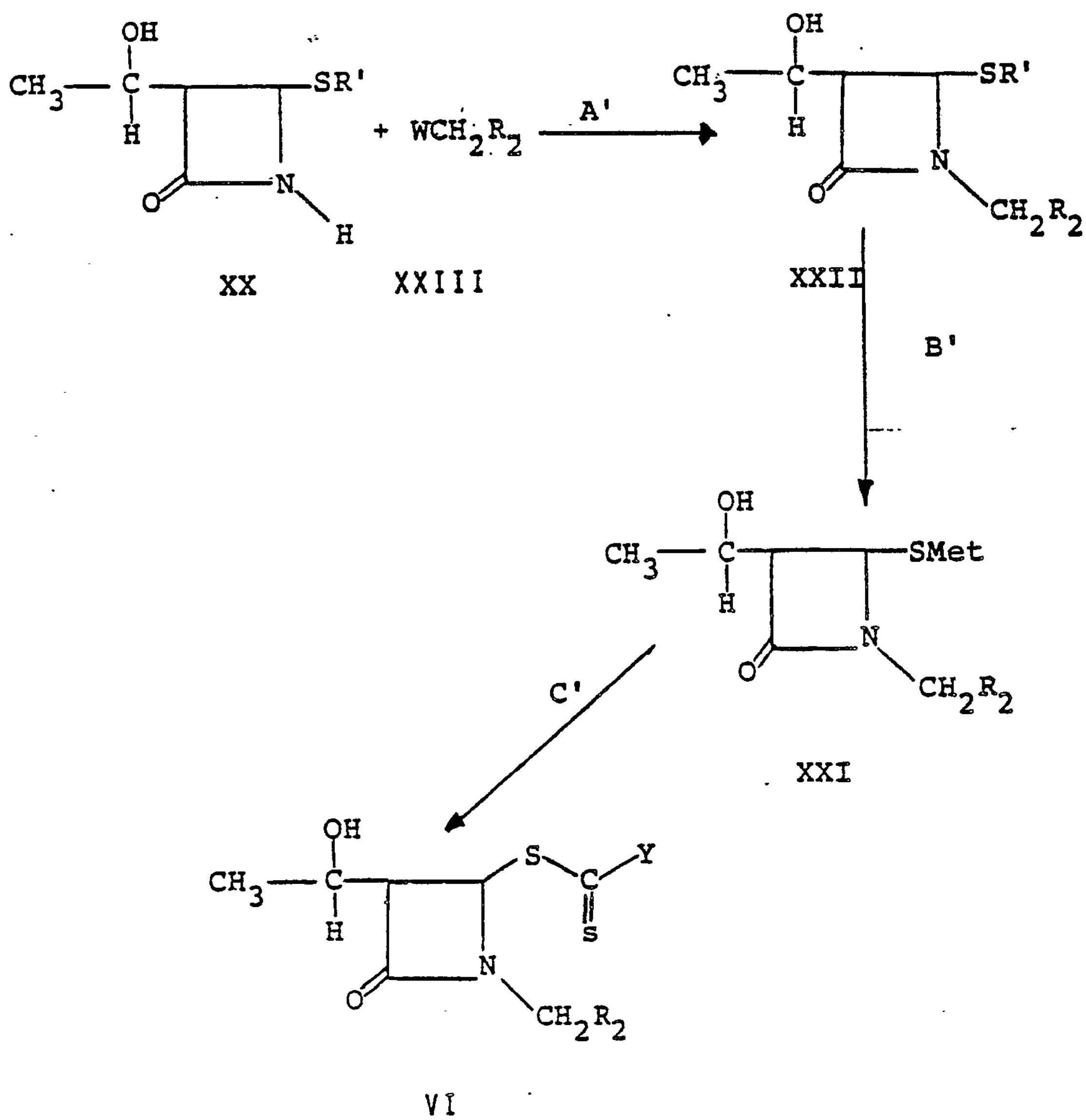


The intermediates of formula VI can be made, for example, by following the reaction scheme 2 as shown in the accompanying flow chart. As in the case of reaction scheme 1 described above, it will be understood that any functional groups in any of the intermediates will be protected if necessary, or desired.

Step A′

The reaction of azetidinone XX with the α-substituted acetate XXIII is preferably carried out in the presence of an acid acceptor, preferably at a temperature in the range from about 15° C to about 30° C.

W is a leaving group preferably tosyl, mesyl, chloro, bromo, iodo or trifluoromethylsulfonyl, most preferably iodo or bromo.

$R_2$ in the acetate XXIII is preferably allylic-oxycarbonyl most preferably allyloxycarbonyl.

If the solvent is also an acid acceptor, e.g. pyridine, no additional acid acceptor is required. Alternatively, an organic solvent which is not an acid acceptor, e.g. acetonitrile may be employed. In these cases, a separate acid acceptor, organic or inorganic must be used. Preferably the reaction is conducted in acetonitrile using cesium carbonate or tetraalkylammonium hydroxide as the acid acceptor.

Step B′ can be carried out by following the procedures described above in connection with step D of

reaction scheme 1.

Step C′ can be carried out by following the procedures described above in connection with the conversion of metal salt II with the thiocarbonyl compound of formula III into thione IV. In this case however, the cyclization (which occurs in reaction scheme 1 with using the malonate diester) does not occur and the thiocarbonyl azetidone intermediate VI is obtained.

Usually the metal salt intermediate of formula XXI will be treated to protect the 5 position hydroxy group (if it is not already protected) before step C′ is carried out. The resulting protected group can be deprotected, if desired, after the cyclization of the compound of formula VI i.e. the deprotection being carried out for example, on the compound of formula V . Conventional hydroxy protection and deprotection procedures can be used as discussed below.

In the preferred form of this process the intermediate of formula XXII is utilized directly in the reaction of step B′, without separation from the reaction mixture in which it is formed (in step A′) and the compound of formula XXI so formed likewise treated, directly, without isolation from the reaction mixture in the procedure of step C′.

If the intermediate of formula XXII formed in step B is treated to protect the 5 position hydroxy group before it is treated in step C′ the protecting procedure can likewise be carried out directly on the intermediate of formula XXI without separating it from the reaction mixture in which it is formed. And likewise the protected intermediate so formed can be treated in step C′ without separation from the reaction mixture.

Similarly the cyclization of intermediate of formula VI and the removal of any 8 position hydroxy protecting group from the resulting compound can be carried out sequentially on intermediates without separating the cyclized product from the reaction mixtures in which they are formed, where the 8-hydroxy group in the resulting penem is protected, this will then be removed by conventional procedures. Subsequent deprotection of the 3 position carboxyl group and formation of a free acid, salt or metabolisable ester can be carried out using the procedures discussed above.

Suitable hydroxy protecting groups are well known in the penem art and methods for their attachement to hydroxy groups are likewise well known. A particularly preferred protecting procedure for instance, for the protection of the 5-hydroxy group in the compounds of formula II and XXI comprises reacting the appropriate intermediate, (e.g. of formula II) having an unprotected hydroxy group, with bis-silylacetamide which readily forms the trimethylsilyl protecting group at the appropriate hydroxy moiety. The protection of the 5-hydroxy moiety in the azetidinone intermediates in the processes discussed above can be carried out without isolation of the intermediate involved. Thus the inert solvent used may be the same as the one used in the preceding step e.g. DMF. Other solvents for example, chloroform and methylene chloride may also be used. Temperatures for the hydroxy protection procedure are typically from about 0°C to about 30°C.

Methods for the removal of a group protecting 5 or 8-hydroxy group are well known in the penem art. Preferably, when the hydroxy protecting group is trimethylsilyl, addition of a mild aqueous acid solution, such as acetic acid, to the solution in which the intermediate to be deprotected is prepared effects removal. Thus, for example, there is no need to isolate a protected derivative of the compound of formula IV before proceeding to deprotect the 8-hydroxy group.

Process (d) involving sulfoxide replacement, is usually carried out in an inert solvent, for example dichloromethane or tetrahydrofuran. The reaction temperature is usually in the range of 0°C to -70°C.

When the thiol compound VIII′ is itself used the reaction is generally carried out in the presence of a base, e.g. an organic base such as diisopropylethylamine or triethylamine, or an inorganic base, for example potassium hydroxide or sodium methoxide.

Alternatively a reactive derivative, for example an alkali metal salt preferably sodium or potassium may be used. The sufoxides of formula VII′ can be obtained by e.g. treating a compound of formula I with a mild oxidizing agent, for example m-chloroperoxybenzoic acid in an inert solvent, for example dichloromethane at between -30°C and 20°C, e.g. 0°C to 5°C.

Such compound of formula I can be prepared, for example by the other methods disclosed herein, or by methods disclosed in our European Patent Specification Publication No. 13662.

Process (e), involving cyclization of the compound of formula IX, is usually carried out analogously to the process described in our European Patent Application, Publication No. 58317.

Thus it is usually carried out in an inert solvent, for example an aromatic hydrocarbon e.g. toluene benzene, aliphatic ethers e.g. diethylether and dipropylether, cyclic ethers e.g. dioxane and tetrahydrofuran, and halogenated hydrocarbons e.g. methylene chloride and chloroform.

In general the cyclization reaction is conducted at temperatures in the range from 20°C to 80°C, usually from 40°C to 60°C for a period of from 6 to 24 hours.

Suitable trivalent organophosphorous compounds are cyclic and/or acyclic trialkylphosphites, triarylphosphites and mixed aryl alkylphosphites or phosphoramides. The preferred trivalent organophosphorous compound is a trialkylphosphite; most preferred is triethylphosphite.

The compounds of formula IX can be obtained by the reaction of a compound of the formula

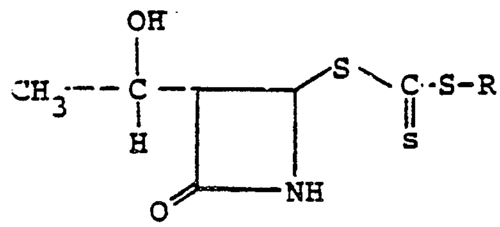

in which R is as defined above
with a reactive derivative e.g. chloride, of an acid of the formula

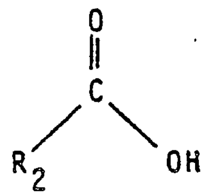

in which $R_2$ is as defined above. This reaction is usually carried out under normal acylating conditions, namely in an inert solvent and in the presence of an organic base, preferably a tertiary amine.

Typical representatives of compounds which can be produced by the methods described above are:

1) (5R,6S,8R)-2-(benzylthio)-6-(1-hydroxyethyl)-penem-3-carboxylic acid
2) (5R,6S,8R)-2-(2-methoxycarbonylmethylthio)-6-(1-hydroxyethyl)-penem-3-carboylic acid
3) (5R,6S,8R)-2-(1-propylthio)-6-(1-hydroxyethyl)-penem-3-carboxylic acid
4) (5R,6S,8R)-2-(N,N-dimethylcarbamimidoylmethylthio)-6-(1-hydroxyethyl)-penem-3-carbooxylic acid
5) (5R,6S,8R)-2-(2,3-dihydroxy-1-propylthio)-6-(1-hydroxyethyl)-penem-3-carboxylic acid
6) (5R,6S,8R)-2-(2-amino-4-thiazolylmethylthio)-6-(1-hydroxyethyl)-penem-3-carboxylic acid
7) (5R,6S,8R)-2-[2-(aminoacetylamino)ethylthio]-6-(1-hydroxyethyl)-penem-3-carboxylic acid
8) (5R,6S,8R)-2-(1-methyl-2-imidazolylmethylthio)-6-(1-hydroxyethyl)-penem-3-carboxylic acid
9) (5R,6S,8R)-2-(N-methylcarbamoylethylthio)-6-(1-hydroxyethyl)-penem-3-carboxylic acid
10) (5R,6S,8R)-2-ethylthio-6-(1-hydroxyethyl)-penem-3-carboxylic acid,
11) (5R,6S,8R)-2-(2-amino-4-thiazolylmethylthio)-6-(1-hydroxyethyl)-penem-3-carboxylic acid

and the alkali metal salts (particularly sodium and potassium salts) and metabolisable esters thereof (particularly pivaloyloxymethyl and phthalidyl esters), corresponding 5R,6R,8S stereoisomers, and diastereomeric mixtures and enantiomeric mixtures comprising the foregoing, and other stereoisomers and stereoisomeric mixtures of the same chemical compounds.

The following preparations, examples and illustrations describe in detail the processes of the present invention, methods for the preparation of the starting material and illustrations of the use of the intermediates produced by the instant process. Throughout these preparations, examples and illustrations, "NMR" denotes nuclear magnetic resonance spectra; "rotation" denotes optical rotation of the compounds in a suitable solvent; "MS" denotes mass spectra; UV denotes ultraviolet spectra; "IR" denotes infrared spectra: Chromatography is performed on silica gel unless otherwise noted. The term "room temperature" refers to about 18° C to about 25° C.

## PREPARATION OF STARTING MATERIALS

## PREPARATION A

(3S,4R,5R)-3-(1-Trichloroethoxycarbonyloxyethyl)-4-(tri phenylmethylthio)azetidin-2-one

To a 250 ml flask add 7.8 grams (0.0223M) of 3-(1-trichloroethoxycarbonyloxyethyl)-4-acetoxyazetidin-2-one, 220ml acetonitrile, 2.6 grams (0.252M) cesium carbonate, and 5.2 grams (0.0188M) triphenylmethanethiol (tritylthiol). After stirring for 5 hours, add an additional 1.0 gram (0.0036M) triphenylmethanethiol and stir the mixture for another one-half hour. After overnight refrigeration filter to remove the solids and remove the solvents under reduced pressure to yield a crude reaction product. Chromatograph this crude product on coarse silica gel eluting with methylene chloride changing to 10% and 20% ethyl acetate/methylene chloride to afford 7.89 grams (3S,4R,5R)-3-(1-trichloroethoxycarbonyloxyethyl)-4-(triphenylmethylthio)azetidin-2-one with spectra as follows:
NMR: = 7.7-7.1,16H; 5.05,1H,m; 4.85,2H,q (J = 18Hz); 4.45,1H,d(J = 1.5Hz); 3.3, 1H,dd(J = 1.5,9Hz); 1.5,3H,d(J = 9Hz).

## PREPARATION B

## DI(TRIMETHYLSILYL)KETOMALONATE

(a) In 100ml of methylene chloride dissolve 22.50 grams 2-trimethylsilylethanol. To this mixture add 20.00 grams triethylamine. Cool to about -20°C., and add a solution of 15 grams of freshly distilled malonyl dichloride in 100ml methylene chloride slowly over a period of one and one-half hours. After the addition is completed, allow the reaction mixture to warm to room temperature and then wash twice with 500ml portions of water, followed by washings with 5% sodium bicarbonate solution until the pH is greater than 9. Dry the solution over anhydrous magnesium sulfate and remove the solvents by evaporation to yield 30.22 grams of the trimethylsilyl diester of malonic acid.

(b) Dissolve diester prepared as described in paragraph (a) in 300ml benzene. Add to this solution 140mg benzoic acid, 17ml benzaldehyde and sufficient piperidine to afford a pH of about 9. Reflux the solution, using a Dean-Stark tube, for 8 hours and then remove the solvents under reduced pressure to afford, as the product, di(trimethylsilylethyl)benzylidinemalonate.

(c) Dissolve the di(trimethylsilylethyl) benzylidene malonate prepared as described in paragraph (b) in 500ml methylene chloride and cool to about 0°C. Bubble ozone into the solution until a distinct blue to blue-green color persists. Discontinue the admission of the ozone and allow the solution to stand for five to ten minutes. Pass nitrogen through the reaction vessel until the excess ozone is completely removed. Add 75 milliliters of dimethyl sulfide and allow the reaction mixture to come to room temperature. Evaporate the solvent and place the resulting oil in an open dish to allow any excess benzaldehyde to oxidize. After standing overnight, dissolve the semi-crystalline mass in methylene chloride and wash it, first with saturated sodium bicarbonate solution, and then with water. Dry the washed methylene chloride solution over anhydrous magnesium sulfate and remove the solvents. The resulting oil/crystalline mass is recrystallized from petroleum ether to afford di(trimethylsilylethyl)ketomalonate.

## PREPARATION C

Preparation of Allyl trimethylsilylethylketomalonate

Add to a 500ml flask 25gm ketomalonic acid 1-1/2 $H_2O$, 250mg p-toluene sulfonic acid, 58gm allyl alcohol, and 200ml benzene. Reflux using a Dean Stark tube for 6-1/2 hours. Remove excess allyl alcohol and benzene by evaporation under vacuum. Wash the residue with $H_2O$, then distill at 2mmHg and collect diallyl ketomalonate as a yellow oil, b.p. 89-92°C, yield 25gm.
Add the thus produced 25gm diallyl ketomalonate to 14.9gm of $(CH_3)_3$ $SiCH_2CH_2OH$, then add 1/2ml of 1,5-diazabicyclo-[4.3.0]non-5-ene (DBN). After 24 hours, wash the resultant mixture with cold 10% phosphoric acid, then with water. Dry the resultant product and distill at 0.4mm Hg to obtain allyl trimethylsilylethylketo

malonate, b.p. 91-100°C., yield 12gm.
NMR: = 0.05, (9H,S); 1.05, (2H,T,J=9 Hz); 4.35, (2H,T,9Hz); 4.70, (2H, D, J=6Hz); 5.25, (2H, M); 5.80, (1H, M)

## PREPARATION D

### 1-METHYL-2-CHLOROMETHYL-IMIDAZOLE

Charge 10 grams of 1-methyl imidazole and 100 ml of a 37% aqueous formaldehyde solution to a 150 ml Parr bomb and heat to 125° C. in an oil bath. Remove the water and evaporate the residue to a gel. Extract the gel in solution with methanol. Remove the methanol. Isolate from a coarse silica column, the product, 1-methyl-2-hydroxymethyl-imidazole, and crystallize from $CCl_4$. Mix 4.4 gram of 1-methyl-2-hydroxymethyl-imidazole with 5.7 ml $SOCl_2$ in 50 ml $CHCl_3$ in a reaction flask. Stir for 18 hours and remove the solvent and excess $SOCl_2$ under vacuum. Evaporate to dryness to recover the product, 1-methyl-2-chloromethyl-imidazole.

## PREPARATION E

### ALLYL-(5R,6S,8R,2'RS)-(ETHANESULFINYL)-6-(1-HYDROXYETHYL)PENEM-3-CARBOXYLATE

Stir a solution of allyl-(5R,6S,8R,2'RS)-2-(ethylthio)-6-(1-hydroxyethyl)penem-3-carboxylate (31.5g) in ethyl acetate (200 ml) and dichloromethane (100 ml) at 0°-5° C. Add a solution of m-chloroperoxybenzoic acid (80-85%; 22g) in ethyl acetate (120 ml) over 0.5 hour. After a further 0.5 hour, add the solution to a stirred mixture of ethyl acetate (150 ml), water (125 ml) and sodium bicarbonate (15 g) and stir rapidly for 15 minutes. Dry the organic phase over $MgSO_4$, evaporate and chromatograph rapidly on silica gel, eluting with 1:1 hexane-ethyl acetate then pure ethyl acetate. Evaporate the product fractions and pump the residue at high vacuum to give the title compound as a thick yellow oil.
PMR($CDCl_3$): δ 1.2-1.6 (m,6H), 3.0-3.35 (m,2H), 3.38 (br.s, 1H, exch by $D_2O$), 3.83 (m, 1H), 4.18 (m, 1H), 4.75 (br.d, J=6.5 Hz), 5.2-5.6 (m, 2H), 5.73 and 5.89 (both d, J=1.5 Hz, total 1H) and 5.8-6.2 (m, 1H).

The compound obtained is a mixture of isomers diastereoisomeric at the oxidized sulfur. The mixture was used in the following examples as both isomers react.

## PREPARATION F

### 2-(N-ALLYLOXYCARBONYLGLYCLAMINO)-ETHANETHIOL

Add pivaloyl chloride (2.4 ml) in $CH_2Cl_2$ (10 ml) to a cooled (0°-5° C.) and stirred solution of N-allyloxycarbonylglycine (3.18 g) and triethylamine (2.8 ml) in dry $CH_2Cl_2$ (50 ml). Stir the mixture at 0°-5° C. for 15 mins. and then add a solution of 2-aminoethanethiol hydrochloride (2.4 g) and triethylamine (2.8 ml) in ethanol (15 ml) and $CH_2Cl_2$ (40 ml). Stir at room temperature for 1 hour, wash the mixture with aqueous 2N-$H_2SO_4$ and aqueous $NaHCO_3$, dry and evaporate. Triturate the resulting solid with ether, filter and dry to give the title compound.
PMR ($CDCl_3$): δ 1.42 (t, J=8 Hz, exch by $D_2O$ 1H), 2.61 (m, 2H), 3.50 (q, J=7 Hz, 2H), 3.88 (d, J=7Hz, 2H), 4.57 (m, 2H), 5.1-5.5 (m, 2H), 5.6-6.2 (m, 2H; 1H exch. by $D_2O$) and 7.0 (br. S, 1H, exch.by $D_2O$).

## EXAMPLE 1

## (5R,6S,8R)-2-ETHYLTHIO-6-(1-HYDROXYETHYL)-PENEM-3-CARBOXYLIC ACID

A. Dissolve 3.0 grams (3S,4R,5R)-3-[1-(2,2,2-trichloroethoxycarbonyloxyethyl)]-4-(triphenylmethylthio)-azetidin-2-one (prepared as described in Preparation A) in 6ml dimethylformamide. To this solution add 2.0 grams di(trimethylsilylethyl) ketomalonate (prepared as described in Preparation B) and molecular sieves. After standing for two days at room temperature, partition the reaction mixture between water and methylene chloride. Separate the organic layer and remove the solvents by rotary evaporation. Purify the crude reaction product so obtained by column chromatography on silica gel eluting with methylene chloride changing to 2% ethyl acetate/methylene chloride, to yield 4.26 grams (3S,4R,5R)-1-[1-hydroxy-1,1-di-(trimethylsilylethoxycarbonyl)methyl]-3-[1-(2,2,2-trichloroethoxycarbonyloxy)ethyl]-4-triphenylmethylthio)-azetidin-2-one, having spectra as follows:

NMR: $\delta$ = 7.5-7.1,15H; 5.05,1H,m; 4.65,2H, s; 4.5,1H,d(J=1.5Hz); 4.2,4H,m; 3.45,1H,dd(J=1.5,7Hz); 1.05,3H,d (J=7Hz); 0.9,4H,m; 0.05,18H.

B. To a solution of 10ml methylene chloride, 2ml pyridine and 1.0 gram calcium carbonate add 4.26 gram (3S, 4R,5R)-1-[1-hydroxy-1,1-di(trimethylsilylethoxycarbonyl)methyl]-3-[1-(2,2,2-trichlororethoxycarbonyloxy)ethyl]-4-triphenylmethylthio)-azetidin-2-one. After placing the mixture in an ice bath add slowly thereto 1.5ml of thionyl chloride. After one-half hour, the reaction is complete. Wash the reaction mixture with sodium bicarbonate solution of pH less than 8 and remove the solvent under reduced pressure. Chromatograph on silica gel using methylene chloride as eluant to obtain 3.48 grams of the product, (3S,4R,5R)-1-[1-chloro-1,1-di(tri-methylsilylethoxycarbonyloxy)methyl]-3[1-(2,2,2-tri-chloroethoxycarbonyloxyethyl]-4-(triphenylmethylthio)azetidin-2-one.

C. Dissolve 3.48 grams of (3S,4R,5R)-1-[1-chloro-1,1-di-(trimethylsilylethoxycarbonyl)methyl]-3-[1-(2,2,2-tri-chloroethoxycarbonyloxyethyl]-4-triphenyl-methylthio)azetidin-2-one in 50ml tetrahydrofuran. To this solution add 15ml water and 8 grams zinc dust. Place the mixture in an ice bath and add 16 grams of ammonium chloride in portions over a period of one hour. After a period of two hours, add 4ml of 100% acetic acid, and then, portionwise, an additional 6 grams of zinc dust. After a further period of one hour, filter the reaction mixture and remove the solvents under reduced pressure. Partition the crude product between water and methylene chloride. Purify using column chromatography on silica gel using as eluant, 1% ethyl acetate/methylene chloride changing to 25% ethyl acetate/methylene chloride to afford 1.644 grams of the desired product, (3S,4R,5R)-1-[1,1-di(trimethylsilylethoxycarbonyl)methyl]-3-(1-hydroxyethyl)-4-(triphenylmethylthio)azetidin-2-one, having spectra as follows:

NMR : = 7.5-7,15H; 4.15,5H; 3.9,1H,s; 3.4,1H,dd(J=1.5,6Hz); 1.05,3H,d (J=6Hz); 0.95,4H,m; 0.05,18H.

D. To a 25 ml flask having a nitrogen atmosphere add 1 ml methanol and 200 mg (0.000289 moles) (3S,4R,5R)-1-[1,1-di(trimethylsilylethoxycarbonyl)methyl]-3-(1-hydroxyethyl)-4-(triphenylmethylthio)azetidin-2-one. Cool the solution to about 0°C, and add 0.025 ml (25 mg, 0.000317 moles) pyridine and 54 mg (0.000317 moles) silver nitrate in 1 ml methanol. Allow the mixture to warm to room temperature, with stirring. After two hours, remove the methanol under high vacuum to afford silver (3S,4R,5R)-3-(1-hydroxyethyl)-1-[di($\beta$-trimethylsilylethyl)-2-malonate]azetidin-2-one-4-thiolate.

E. Dissolve silver (3S,4R,5R)-3-(1-hydroxyethyl)-1-[di($\beta$-trimethylsilylethyl)-2-malonate]-azetidin-2-one-4-thiolate in 2 ml methylene chloride and to this solution added 68 mg of 1,1-thiocarbonyldiimidazole. Stir for another 1.5 hour, and then directly apply the reaction mixture to a chromatography column of silica gel. Elute with methylene chloride to afford the desired product, (5R,6S,8R)-2-thione-3,3-di-(trimethylsilylethoxycarbonyl)-6-(1-hydroxyethyl)-penem, having spectra as follows:

NMR: = 5.7,1H,d(J=1Hz); 4.2,5H,m; 3.65 1H,dd(J=1,8Hz); 1.3,3H,d(J-8Hz); 0.95,4H,m; 0.05,18H.

F. 61 miligrams of (5R,6S,8R)-2-thione-3, 3-di (trimethylsilylethoxycarbonyl)-6-(1-hydroxyethyl)penam is dissolved in 5ml tetrahydrofuran and 2 equivalents of tetrabutylammonium fluoride in 10ml tetrahydrofuran is slowly added at room temperature. Thin layer chromatography (silica, 10% ethyl acetate/methylene chloride) showed the immediate presence of the decarboxylated compound (5R,6S,8R)-2-thione-3-(trimethylsilylethoxycarbonyl)-6-(1-hydroxyethyl)-penam, which exists in equilibrium with (5R,6S,8R)-2-thiol-3-(trimethylsilylethoxycarbonyl)-6-(1-hydroxyethyl)penem.

G. To the solution of (5R,6S,8R)-2-thione-3-(trimethylsilylethoxycarbonyl)-6-(1-hydroxyethyl)penam and (5R,6S,8R)-2-thiol-3-(trimethylsilylethoxycarbonyl)-6-(1-hydroxyethyl)penem produced in the above step F add 2 ml of ethyl iodide. Stir at room temperature for about 15 minutes then partition between water and ethyl acetate. Separate the organic layer and remove the solvents by rotary evaporation to yield the desired

product, (5R,6S,8R)-β-(trimethylsilyl)ethyl-2-ethylthio-6-(1-hydroxyethyl)penem-3-carboxylate, having spectra as follows:

NMR: = 5.7,1H,d(J=1.5Hz); 4.2,5H,m; 3.7,1H,dd(J=1.5,7Hz); 3,2H,m; 1.4-0.9,8H; 0.05,9H.

H. Dissolve 40 miligrams of (5R,6S,8R)-β-(trimethyl-silyl)ethyl-2-ethylthio-6-(1-hydroxyethyl)penem-3-carboxylate in 1ml tetrahydrofuran and to this slowly add one equivalent of tetrabutylammonium fluoride in 2ml tetrahydrofuran at room temperature. After 15 minutes, the reaction is complete as shown by thin layer chromatography. Acidify with phosphoric acid to a pH not below 2, and thereafter purify to afford the desired product, (5R,6S,8R)-2-ethylthio-6-(1-hydroxyethyl)-penem-3-carboxylic acid, identifiable by spectra and bioautogram with authentic (5R,6S,8R)-2-ethylthio-6-(1-hydroxyethyl)penem-3-carboxylic acid.

## EXAMPLE 2

Repeat the procedures detailed in Example 1, steps A to H, but use methyl iodide in place of the ethyl iodide utilized in step G to obtain (5R,6S,8R)-2-methylthio-6-(1-hydroxyethyl)penem-3-carboxylic acid.

## EXAMPLE 3

Repeat the procedure of Example 1 but use n-propyliodide in place of the ethyl iodide of step G to obtain (5R,6S,8R)-2-n-propylthio-6-(1-hydroxyethyl)penem-3-carboxylic acid.

## EXAMPLE 4

Repeat the procedure of Example 1 but use isopropyl iodide in place of the ethyl iodide of step G to obtain (5R,6S,8R)-2-isopropylthio-6-(1-hydroxyethyl)penem-3-carboxylic acid.

## EXAMPLE 5

Repeat the procedure of Example 1 but using ethylene and, as a radical initiator, AIBN[2,2′-azobis (2-methyl-propionitrile)] in step G in place of ethyl iodide, thereby affording (5R,6S,8R)-2-ethylthio-6-(1-hydroxyethyl)penem-3-carboxylic acid.

## EXAMPLE 6

Repeat the procedure of Example 5 but using methyl vinyl ketone in place of ethylene thereby affording (5R,6S,8R)-2-(2-oxo-4-butylthio)-6-(1-hydroxyethyl)penem carboxylic acid.

## EXAMPLE 7

### Preparation of Allyl-(5R,6S,8R)-2-ethylthio-6-(1-hydroxyethyl)penem-3-carboxylate

A) Preparation of (3S,4R,5R)-1-[1-hydroxy-1-allyloxycarbonyl-1-trimethylsilylethoxycarbonyl-methyl]-3-[1-(2,2,2-trichloroethoxycarbonyloxyethyl)]-4-(triphenylmethylthio)azetidin-2-one

Add 100mg of (3S,4R,5R)-3-[1-(2,2,2-trichloroethoxycarbonyloxyethyl -4-(triphenylmethylthio)-azetidin-2-one (prepared as described in Preparation A) and 0.2ml of dimethylformamide to a dry vial. Add 45mg of allyl trimethylsilylethylketomalonate (prepared as described in Preparation C), 0.0014ml of pyridine and 0.0014ml of triethylamine to the system. After standing at room temperature for 50 minutes, remove the solvent by stripping to give the title product.

B) Preparation of (3S,4R,5R)-1-[1-allyloxycarbonyl-1-chloro-1-trimethylsilylethoxycarbonylmethyl]-3-[1-(2,2,2-trichloroethoxycarbonyloxy)ethyl]-4-(triphenylmethylthio)-azetidin-2-one

Add 4.26 mg of (3S,4R,5R)-1-[1-hydroxy-1-allyloxycarbonyl-1-trimethylsilylethoxycarbonyl-methyl]-3-[1-(2,2,2-trichloroethoxycarbonyloxy)ethyl]-4-(triphenylmethylthio)-azetidin-2-one to a solution of 10 ml of methylene chloride, 2ml pyridine and 1gm of calcium carbonate. Cool the system to 0°-5°C., by placing the system in an ice bath. After cooling, slowly add 1.5ml of thionyl chloride. After 25 minutes, the reaction is complete. Wash the reaction mixture with sodium bicarbonate solution of pH less than 8 and remove the solvent by stripping. Chromatograph the residue on silica gel using methylene chloride as the eluant to afford 3.48gm of the title compound.

C) Preparation of (3S,4R,5R)-1-[1-allyloxycarbonyl-1-trimethylsilylethoxycarbonyl-methyl]-3-(1-hydroxyethyl)-4-(triphenylmethylthio)-azetidin-2-one

Dissolve 3.48gm of (3S,4R,5R)-1-[1-allyloxycarbonyl-1-chloro-1-trimethylsilylethoxycarbonyl-methyl]-3-[1-(2,2,2-trichloroethoxycarbonyloxy)ethyl]-4-(triphenylmethylthio)-azetidin-2-one in 50ml of tetrahydrofuran. To the system add 15ml of water and 8gm of zinc dust. Place the system in an ice bath and add 16gm of ammonium chloride in portions over 1 hour. Stir the solution at 0°-5°C., for an additional 2 hours and then add 4ml of glacial acetic acid and, portionwise, an additional 6gms of zinc dust. Continue the reaction for an additional 1 hour, filter and remove the solvent by stripping. Dissolve the crude product in methylene chloride and wash the organic solution with water. Purify the crude product by chromatography on silica gel using as eluant 1% ethylacetate (methylene chloride changing to 25% ethylacetate) to afford 1.64gm of title compound.
NMR: = .05, (5, 9H); 1.05, (m, 2H); 1.15, (D, 3H, J=6); 2.2, (5, 1H); 3.38, (DD, 1H); 3.7, (m); 4.2, (m); 4.5, (m); 5.2, (m, 2H); 5.8, (m, 1H).

D) Preparation of Silver- (3S,4R,5R)-3-(1-hydroxyethyl)-1-allyloxycarbonyl-1-trimethylsilylethoxycarbonyl-methyl-azetidin-2-one-4-thiolate

To a 50 ml flask having a nitrogen atmosphere add 5 ml of methanol and 1 gm (0.00158 moles) of (3S,4R,5R)-1-[1-allyloxycarbonyl- 1-trimethylsilylethoxycarbonylmethyl]-3(1-hydroxyethyl)-4-triphenylmethylthio-azetidin-2-one. Cool the solution to about 0°C and then add 0.14 ml of pyridine and 1.74 ml of methanol containing 294 mg (0.00173 moles) of silver nitrate. Stir the system at about 0°C for 1 hour and then allow the system to warm to room temperature. After 2 hours of stirring at room temperature, add an additional 0.2 ml of methanol containing 34 mg of silver nitrate (0.0002 moles) to the system and continue the reaction for an additional 1 hour. Stop the reaction and remove the methanol by stripping. Dissolve the residue in methylene chloride and wash the organic solution twice with water, then with brine. Dry the organic solution over anhydrous sodium sulfate, filter and remove the methylene chloride by stripping to give the title compound.

D′)

Repeat the procedure set forth in step D but replacing the silver nitrate with an equivalent quantity of 1) thallic nitrate, and 2) cupric nitrate to obtain respectively
thallium (3S,4R,5R)-3-(1-hydroxyethyl)-1-allyloxycarbonyl-1-trimethylsilylethoxycarbonyl-methyl-azetidin-2-one-thiolate;

and
copper (3S,4R,5R)-3-(1-hydroxyethyl)-1-allyloxycarbonyl-1-trimethylsilylethoxycarbonyl-methyl-azetidin-2-one-4-thiolate.

## PREPARATION D"

D")

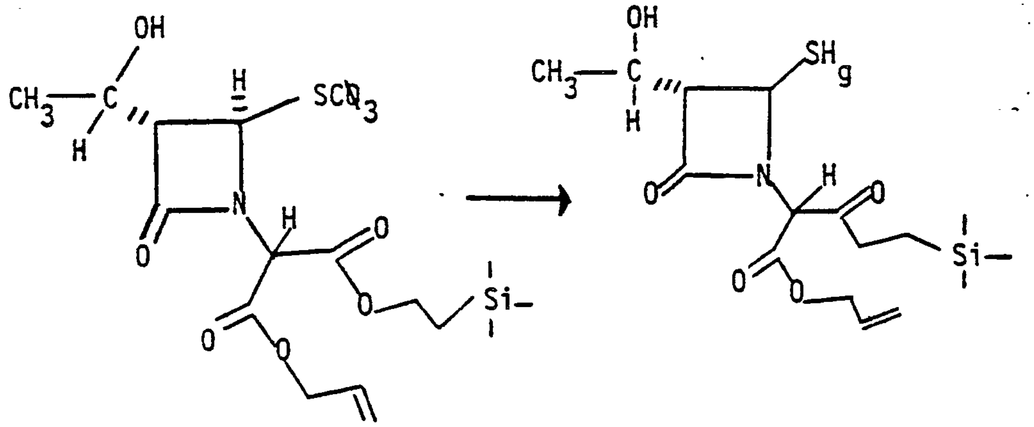

(I) (II)

Charge a 50 ml flask, under nitrogen, with 3.98 g (0.00631 M) compound I, in 10 ml methanol, cool to 0° C. and add a solution of 2.16 g (0.0631 M) $Hg(NO_3)_2.H_2O$ and 0.545 g (0.0069 M) pyridine in methanol. Stir for 3 hours at room temperature and remove methanol under reduced pressure. Dissolve the residue in methylene chloride and wash the organic solution twice with water, then with brine. Dry the organic solution over anhydrous sodium sulfate, filter and remove the methylene under reduced pressure to give the compound II.

E) Preparation of Silver (3S,4R,5R)-3-(1-trimethylsilyloxyethyl)-1-allyloxycarbonyl-1-trimethylsilylethoxycarbonyl-methyl azetidin-2-one-4-thiolate

Dissolve the entire amount of silver (3S,4R,5R)-3-(1-hydroxyethyl)-1-allyloxycarbonyl-1-trimethylsilylethoxycarbonyl-methyl-azetidin-2-one-4-thiolate obtained from step (D) above in 10 ml of anhydrous methylene chloride. Add 0.783 ml (0.00316 moles) of bis-trimethylsilyl acetamide to the system. Stir the system at room temperature for 15 minutes to yield the title compound.

E′)

Repeat the procedure of step E but replacing the silver salt by the thallium, copper and mercury azetidone salts obtained in steps D′ and D″ to obtain respectively thallium (3S,4R,5R)-3-(1-trimethylsilyloxyethyl)-1-allyloxycarbonyl-1-trimethylsilylethoxycarbonyl-methyl azetidin-2-one-4-thiolate,
copper (3S,4R,5R)-3-(1-trimethylsilyloxyethyl)-1-allyloxycarbonyl-1-trimethylsilylethoxycarbonyl-methyl-azetidin-2-one-4-thiolate and
mercury (3S,4R,5R)-3-(1-trimethylsilyloxyethyl)-1-allyloxycarbonyl-1-trimethylsilylethoxycarbonyl-methyl-azetidin-2-one-4-thiolate.

F) Preparation of (5R,6S,8R)-2-thione-3-allyloxycarbonyl-3-trimethylsilylethoxycarbonyl-6-(1-trimethylsilyloxyethyl)penam

After the completion of step (E) and to the same solution, add 619 mg (0.00316 moles) of 90% thiocarbonyl-diimidazole to the system. Stir the system at room temperature for 20 hours and then filter the

solution. Remove the methylene chloride by stripping. Chromatograph the crude product on silica gel eluting with 30% cyclohexane / methylene chloride changing to methylene chloride to afford 704 mg of the title compound.

F′)

Repeat step F but using the reaction solutions obtained in step E′, to afford the title compound.
NMR: = 6.2-5.6, m, 1H; 5.65, d (J = 1.5 Hz), 1H; 5.5-5.1, (m), 2H; 4.7, d (J = 5.5Hz), 2H; 4.5-4.1, m, 3H; 3.62, d,d (J = 1.5, 4Hz), 1H; 1.28, d (J = 6Hz), 3H; 1.2-0.85, m, 2H; 0.2-0, m, 18H.

G) Preparation of (5R,6S,8R)-2-thione-3-allyloxycarbonyl-3-trimethylsilylethoxycarbonyl-6-(1-hydroxyethyl)-penam

To a 25ml flask add 100mg of (5R,6S,8R)-2-thione-3-allyloxycarbonyl-3-trimethylsilylethoxy-carbonyl-6-(1-trimethylsilyloxyethyl)penam, 1ml of tetrahydrofuran 0.05ml of water and 0.05ml of acetic acid. Stir the system at room temperature for 12 hours. Add ethyl acetate to the solution and wash the organic phase with sodium bicarbonate solution, water and then brine. Dry the organic phase over anhydrous sodium sulfate, filter and remove the solvent by stripping to give the title compound.
NMR; = 6.15-5.6, m, 1H; 5.69, d(J-2Hz), 1H; 5.55-5.12, m, 2H; 4.8-4.6, M, 2H; 4.5-4.0, m, 3 H; 3.67, d, d-(J = 2, 7Hz), 1H; 2.8-2.3, m, 1H; 1.37, d(J = 6.Hz), 3H; 1.2-0.8, m, 2H; 0.3-0, m, 9H.

H) Preparation of (5R,6S,8R)Allyl-2-thiol-6-(1-hydroxyethyl)penem-3-carboxylate

To 7.7mg of (5R,6S,8R)-2-thione-3-allyl-oxycarbonyl-3-trimethylsilylethoxycarbonyl-6-(1-hydroxyethyl)-penam in 1ml of tetrahydrofuran slowly add at room temperature 2 equivalents of tetrabutylammoniumfluoride in 40ml of tetrahydrofuran. Thin layer chromatography (silica gel, 10% ethylacetate/methylene chloride) shows the immediate presence of the decarboxylated compound (5R,6S,8R)allyl-2-thiol-6-(1-hydroxyethyl)penem-3-carboxylate, which exists in equilibrium with (5R,6S,8R)-allyl-2-thione-6-(1-hydroxyethyl)penam-3-carboxylate.
NMR: = d 5.85, d (J = 1Hz), 1H; 5.8, m, 1H; 5, 1H; 5.4-5.1, m, 2H; 4.7, 2H; 4.25, M, 1H; 3.65, d, d (J = 1, 1Hz), 1H; 2.1, 1H; 1.35, d (J = 7Hz) 3H.

I) Preparation of Allyl-(5R,6S,8R)-2-ethylthio-6-(1-hydroxyethyl)penem-3-carboxylate

To the solution of a (5R,6S,8R) allyl-2-thiol-6-(1-hydroxyethyl)penem-3-carboxylate (5R,6S,8R) : allyl-2-thione-6-(1-hydroxyethyl)penem-3-carboxylate equilibrium mixture produced in step (H) above add 0.016ml of ethyl iodide and 16mg sodium bicarbonate in 0.5ml water. Stir the system at room temperature for 15 minutes then add 25ml of ethyl acetate. Wash the organic solution with water, dry the organic phase with anhydrous sodium sulfate, filter and remove the solvent by stripping to yield the title compound.

## EXAMPLE 8

Preparation of Allyl-(5R,6S,8R)-2-thiol-6-(1-hydroxy-ethyl)penem-3-carboxylate and Allyl (5R,6S,8R)-2-thione 6-(1-hydroxyethyl)penam-3-carboxylate

A) Preparation of (3S,4R,5R)-1-allyloxycarbonylmethyl)-3-(1-hydroxyethyl)-4-(triphenylmethylthio)azetidin-2-one

Add 3gm of (3S,4R,5R)-3-(1-hydroxyethyl)-4-(triphenylmethylthio)azetidin-2-one to 10ml of acetonitrile containing 0.286gm of cesium carbonate. Add 0.2gm of α-iodo allyl acetate to the system. Stir the system

at room temperature for 16 hours. Dilute with ether (50ml), filter and wash the ether layer with 1% aqueous phosphoric acid, followed by water. After drying over sodium sulfate remove solvent to give a foamy solid. NMR: = 8.4, 1H, s; 7.65, 1H, d(J = 1Hz); 7.05, 1H d(J = 1Hz); 5.95, 1H, d (J = 2Hz); 5.8, 1H, m; 5.45-5.1, 2H, m; 4.3, 1H, m; 4.1, 2H, Q(J = 16Hz); 3.5, d d(J = 2,6); 1.35; 3H, d(J = 6Hz).

B) Preparation of Silver (3S,4R,5R)-3-(1-hydroxyethyl)-1-allyloxycarbonylmethyl-azetidin-2-one-4-thiolate

To a 50ml flask having a nitrogen atmosphere add 10ml of methanol and 460mg of (3S,4R,5R)-1-(allyloxycarbonyl)-3-(1-hydroxyethyl)-4-(triphenylmethylthio)azetidin-2-one. To this system add 160mg silver nitrate and 0.15ml of pyridine. Stir the system at 20°C for 1 hour. Stop the reaction and remove the methanol by stripping to give the title compound.

B′)

Repeat the procedure of step B but replacing the silver nitrate with an equivalent quantity of -

1) thallic nitrate,
2) cupric nitrate and
3) $Hg(NO_3)_2.H_2O$

to obtain respectively thallium (3S,4R,5R)-3-(1-hydroxyethyl)-1-allyloxycarbonylmethyl-azetidin-2-one-4-thiolate,
copper (3S,4R,5R)-3-(1-hydroxyethyl)-1-allyloxycarbonylmethyl-azetidin-2-one-4-thiolate, and
mercury (3S,4R,5R)-3-(1-hydroxyethyl)-1-allyloxycarbonylmethyl-azetidin-2-one-4-thiolate.

C) Preparation of Silver (3S,4R,5R)-3-(1-trimethylsilyloxyethyl)-1-allyloxycarbonylmethyl-azetidin-2-one-4-thiolate

Add the entire amount of silver (3S,4R,5R)-3-(1-hydroxyethyl)-1-allyloxycarbonyl-methylazetidin-2-one-4-thiolate produced in step (B) above to 25ml of methylene chloride. To this system add 1.1ml of bis-silylacetamide. Stir the system at room temperature for 15 minutes to give the title compound.

C′)

Repeat the procedure of step C but replacing the silver salt with the respective thallium, copper and mercury salts obtained in step B′ to afford respectively thallium (3S,4R,5R)-3-(1-trimethylsilyloxyethyl)-1-allyloxycarbonylmethyl-azetidin-2-one-4-thiolate, copper (3S,4R,5R)-3-(1-trimethylsilyloxyethyl)-1-allyloxycarbonylmethyl-azetidin-2-one-4-thiolate, and mercury (3S,4R,5R)-3-6)-trimethylsilyloxyethyl)-1-allyloxycarbonylmethyl-azetidin-2-one-4-thiolate.

D) Preparation of (3S,4R,5R)-1-(allyloxycarbonylmethyl)-3-(1-trimethylsilyloxymethyl)-4-(1′-imidazolylthiocarbonylthio)azetidin-2-one

After completion of step (C) above and to the same solution add 350mg of thiocarbonyldiimidazole. Stir the system at room temperature for 3 hours. Filter the solution and wash the precipitate with methylene chloride. Collect the filtrate and remove the methylene chloride by stripping. Chromatograph the residue on silica gel eluting with 20% ethyl acetate/methylene chloride to yield 335mg of the title compound.

D′)

Repeat the procedure of step D but using the respective thallium, copper and mercury salts produced in step C′ to afford in each case the title compound.

E) Preparation of (5R,6S,8R) allyl-2-thiol-6-(1-trimethylsilyloxymethyl)penem-3-carboxylate and (5R,6S,8R) allyl-2-thione-6-(1-trimethylsilyloxymethyl)penam equilibrium mixture

Add 170mg of (3S,4R,5R)-1-(allyloxycarbonylmethyl)-3-(1-trimethylsilyloxymethyl)-4-(1′-imidazolylthiocarbonylthio)azetidin-2-one to 40ml of anhydrous tetrahydrofuran under a nitrogen atmosphere. Cool the system to -78°C. and then add 0.6ml of 1 M lithium di-(trimethylsilyl) amine in hexane dropwise to the system. Stir the system at -78°C. for 5 minutes. Add 0.2ml of acetic acid to the system. Dilute the system to 200ml with methylene chloride. Wash the organic solution with water, aqueous sodium bicarbonate solution and again with water. Purify the product by chromatography by rapidly eluting the sample through silica gel with 5% ethyl acetate/methylene chloride to afford 125mg of the desired products and the desilylated products.

F) Preparation of (5R,6S,8R) Allyl-2-thiol-6-(1-hydroxyethyl)penem-3-carboxylate and (5R,6S,8R) Allyl-2-thione 6-(1-hydroxyethyl)penam equilibrium mixture

To a 25ml flask add the entire mixture produced in step (E) along with 5ml of tetrahydrofuran, 1ml of water and 1ml of acetic acid. Stir the system at room temperature for 2 hours. Add ethyl acetate to the solution and wash the organic phase with sodium bicarbonate solution, water and then brine. Dry the organic phase over anhydrous sodium sulfate, filter and remove the solvent by stripping to give the title compound.

Thereafter the product of step F can be treated, if desired by the procedure described in step I of the Example 7 to yield allyl (5R,6S,8R)-2-ethylthio-6-(1-hydroxyethyl)penem 3-carboxylate.

## EXAMPLE 9

Preparation of (5R,6S,8R) Allyl-2-(2′,2′,2′-trifluoroethyl)-6-(1-hydroxyethyl)penem-3-carboxylate

1. Dissolve 0.735ml pyridine in 25ml dry toluene and cool to -20°C. under nitrogen. Add 1.45ml trifluoromethanesulfonic anhydride followed by 0.703ml 2,2,2-trifluoroethanol and allow to warm to room temperature. Wash the resultant residue with water, dry with anhydrous sodium sulfate and distill, collecting all fractions with a boiling point less than 100°C to obtain trifluoromethyl 2,2,2-trifluoroethylsulfonate.

2. Add (5R,6S,8R) allyl-2-thiol-6-(1-hydroxyethyl)penem-3-carboxylate, (5R,6S,8R) allyl-2-thione-6-(1-hydroxyethyl)penam-3-carboxylate equilibrium mixture (produced by following the procedure of Example 7 up to step H)
to 3ml of tetrahydrofuran and 5ml of trifluoromethyl 2,2,2-trifluoroethylsulfonate. Add 1 equivalent of potassium carbonate (powder) to the system. Maintain the reaction mixture at room temperature for 1-1/2 hours and then store the solution in a refrigerator overnight. Remove the solution from the refrigerator and stir at room temperature for 1 hour. Filter the solution and wash with methylene chloride/2% phosphoric acid. Remove the solvent by evaporation. Dissolve the residue in warm 1:1 chloroform:petroleum ether and cool. The product crystallizes from solution to yield 168 mg of the title compound.

## EXAMPLE 10

Sodium-(5R,6S,8R)-2-(2,3-dihydroxy-1-propylthio)-6-(1-hydroxyethyl)-penem-3-carboxylate

A.

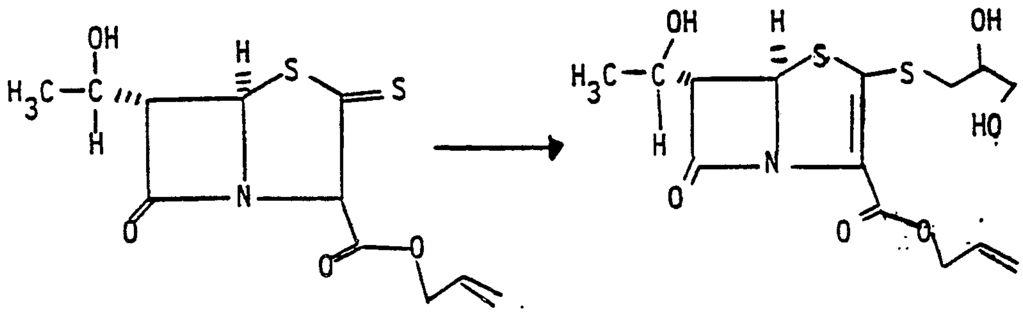

(I) (II)

Add to 100 ml flask 400 mg (0.000696 M) of an equilibrium mixture of thione I with its corresponding thiol tautomer (Example 7, step H), 10 ml distilled tetrahydrofuran, 154 mg (0.00208 M) (3 eq.) of glycidol and 25 mg potassium carbonate. Stir until thin layer chromatography indicates the absence of starting material. Acidify with 10% phosphoric acid up to pH7, remove the tetrahydrofuran on a rotary evaporator and chromatograph the residue on a coarse silica gel column with 100% ethyl acetate to yield, after isolation, 160 mg of compound II (61% yield).

B. To a 50 ml flask add, under a nitrogen atmosphere a solution of the 160 mg of compound II obtained in step A in 2 ml ethyl acetate, 73 mg (0.000442 M) sodium-ethyl-2-hexanoate, 10 mg triphenylphosphine and 5 mg $Pd(Ph_3)P_4$. Stir the reaction mixture for 3 hours, re-frigerate overnight, add water and extract the product on a HPLC column with 100% $H_2O$, to yield, after isolation 32 mg of the title compound as a white amorphous solid.

## EXAMPLE 11

Sodium (5R,6S,8R)-2-(2,3-dihydroxy-1-propylthio)-6-(1-hydroxyethyl)-penem-3-carboxylate.

A. (3S,4R,5R)-3-(1-trichloroethoxycarbonyloxyethyl)-4-[(2,3-dihydroxy-1-propylthio)carbonthioylthio]-azetidin-2-one

To a solution of ethanol (50 ml) containing 1-N sodium hydroxide (20 ml) and 2,3-dihydroxypropanethiol (2 g) add carbon disulfide (4 ml) dropwise, stir ten minutes, then add dropwise to a solution of (3S,4R,5R)-4-[1-(2-methoxy-1,2-dioxoethyl)]-3-(1-trichloroethoxycarbonyloxyethyl)-4-chloroazetidin-2-one (4.1 g) in ethanol. Stir the reaction mixture until t.l.c. analysis indicates no starting compound is present (about 4 hours) then dilute with ethyl acetate,wash the solution with saturated sodium chloride, dry the solution over magnesium sulfate, evaporate, and chromatograph the resulting residue on silica gel (40 g) eluting with 30% etherhexane. Combine the like elutes as determined by the t.l.c. and evaporate to a residue which is the title compound. I.R. = 5.65 $\mu$;
NMR: 5.5ppm (1H,d,J=2 cps)
3.4ppm (1H,q,J=8 and 2 cps)

B. Allyl-(5R,6S,8R)-6-(1-trichloroethoxy-carbonyloxyethyl)-2-(2,3-dihydroxy-1-propylthio)-penem-3-carboxylate

To a solution of (3S,4R,5R)-3-(trichloroethoxycarbonyloxyethyl)-4-[carbonothioylthio]-azetidine-2-one (0.7 g) in methylene chloride (6 ml) cooled to 10°C, add with stirring, calcium carbonate (0.6 g) followed by allyloxalyl chloride (0.263 g, 1.2 eq.). Add dropwise a solution of diisopropylethylamine (0.32 ml, 1.2 eq.) in methylene chloride (1 ml), during 5 minutes while maintaining the temperature at 10°-15°C. After TLC shows no starting compound (15 mins) at 15°C., transfer the mixture to a separatory funnel using ethanol-free chloroform. Wash twice with ice/water, filter to remove excess calcium carbonate, dry over anhydrous sodium sulfate, and transfer to a 100 ml 3-neck flask. Adjust the volume of the solution to approximately 50 ml with chloroform and heat at reflux temperature while adding a solution of triethylphosphite (0.6 ml, 2 eq.) in chloroform (20 ml) over a 3 hour period. Reflux the mixture for an additional 18 hours, evaporate and chromatograph on 14 g silica gel, eluting with 25% ether-hexane, and evaporate the combined like elutes to obtain a residue (420 mg) comprising the title compound. Purify by crystallization from ether-hexane to obtain the title compound in crystalline form.

C. Allyl-(5R,6S,8R)-6-(1-hydroxyethyl)-2-(2,3-dihydroxy-1-propylthio)-2-penem-3-carboxylate

Dissolve about 1.6 g of the product prepared according to the process of step B, in 15 ml of tetrahydrofuran, 1.5 ml of water and 1.5 ml of acetic acid at 0°-5°C. with stirring. Add 2.0 g of zinc dust and stir until thin layer chromatography indicates only a trace of starting material. Filter the reaction mixture, wash solids with ethyl acetate, combine the organic solvents and wash successively with 10% aqueous tartaric acid, water and with aqueous sodium bicarbonate solution. dry the solvent phase over magnesium sulfate and concentrate to a residue. Crystallize the residue from ether-hexane.

D. Sodium-(5R,6S,8R)-6-(1-hydroxyethyl)-2-(2-2,3-dihydroxy-1-propylthio)-penem-3-carboxylate

Dissolve about 6.4 g of the product prepared according to steps A - C in 190 ml of methylene chloride and add 5.32 g of sodium 2-ethylhexanoate in 190 ml of ethyl acetate. Add a mixture of 0.46 gm of triphenylphosphine and 0.46 g of tetrakis (triphenylphosphine)palladium. Stir for 1.5 hours and centrifuge. Wash the precipitate with ethyl acetate and dry at high vacuum to give the title compound as a white amorphous solid.

## EXAMPLE 12

Sodium-(5R,6S,8R)-2-(2,3-dihydroxypropylthio)-6-(1-hydroxyethyl)-penem-3-carboxylate

A. To a solution of 0.20 g of an equilibrium mixture of allyl-(5R,6S,8R)-6-(1-hydroxyethyl)-2-thione and allyl-(5R,6S,8R)-6-(1-hydroxyethyl)-2-thiol-penem-3-carboxylate and diisopropylethylamine (0.15 g) in dry acetonitrile (4 ml) add 2,3-dihydroxy-1-bromopropane (0.2 g). After about 1 hour at 25°C. dilute with aqueous tartaric acid and dry over magnesium sulfate. Evaporate and isolate the residue from dichloromethane to give allyl (5R,6S,8R)-6-(1-hydroxyethyl)-2-(2,3-dihydroxypropylthio)-penem-3-carboxylate.

B. Treat 160 mg of the product compound from step A to exactly the same procedure as in Example 10B to yield after isolation 32 mg of the title compound.

## EXAMPLE 13

A. Prepare allyl-(5R,6S,8R,2′RS)-2-(ethanesulfinyl)-6-(1-hydroxyethyl)-penem-3-carboxylate according to the procedure described in Preparation E.

B. Stir a solution of the product of step A (0.50 g) and 2,3-dihydroxypropylthiol (0.30 g) at about 0° to 5°C in dichloromethane and add thereto diisopropylethylamine (0.2 g). After 5 minutes was the solution with dilute aqueous tartaric acid, dry, evaporate and purify the residue by preparative TLC to obtain allyl-(5R,6S,8R)-2-(2,3-dihydroxy-1-propylthio)-6-(1-hydroxyethyl)-penem-3-carboxylic acid.

C. Treat 160 mg product obtained in step B by the procedure described in example 10, step B to obtain 32 mg sodium (5R,6S,8R)-2-(2,3-dihydroxy-1-propylthio)-6-(1-hydroxyethyl)-penem-3-carboxylic acid.

## EXAMPLE 14

(5R,6S,8R)-2-(1-Methyl-2-imidazolylmethylthio)-6-(1-hydroxyethyl)-penem-3-carboxylic acid

A. (5R,6S,8R)-Allyl-2-(1-methyl-2-imidazolylmethylthio)-6-(1-hydroxyethyl)-penem-3-carboxylate

Charge to a 250 ml flask under nitrogen 1.38 gram of the thione-thiol equilibrium mixture of Example 7, step H, 50 ml of tetrahydrofuran (THF) and 1.2 gram of 1-methyl-2-chloromethyl-imidazole (preparation D). Cool to 0° C. Add dropwise over 3 minutes 1.15 gram of $NaHCO_3$ as a 10% aqueous solution. Stir for about 45 minutes and let stand for one hour at 0° C. Remove the THF solvent and recover the title product on a silica column.

NMR - ($CDCl_3$) $\delta$ = 6.95, 1H,s; 6.86, 1H, s; 5.9, 1H, m; 5.73, 1H, d; 5.32, 2H, m; 4.7, 2H, m; 4.3, 2H, s; 4.2, 1H, m; 3.7, 1H, dd (J = 1.5, 6Hz); 3.68, 3H, s; 1.3, 3H,d (J = 6 Hz).

B.

Dissolve 1.05 grams of the product of step A in 40 ml of the ethyl acetate. React at room temperature with about 200 mg Pd° reagent, 200 mg triphenyl phosphine and 1.5 ml hexanoic acid. Extract the resulting title compound with water. Increase the yield by dissolving unreacted starting material in 15 ml $CH_2Cl_2$, 1 ml hexanoic acid, 0.3 ml pyridine, 1.50 mg triphenyl phosphine and about 100 mg Pd° for 1/2 hour. Extract the resulting title compound with water and combine with the previous extract - the product has
NMR - ($D_2O$) = 7.3, 1H, s; 7.27, 1H, s; 5.6, 1H, d(J = 1.6 Hz); 4.4, 1H, m; 3.85, 1H, dd(J = 1.5, 6Hz); 3.81, 3H, s; 1.24, 3H, d(J = 6 Hz).

EXAMPLE 15

A. Prepare allyl-(5R,6S,8R,2′RS)-2-(ethanesulfinyl)-6-(1-hydroxyethyl)-penem-3-carboxylate according to the procedure described in preparation E.

B. Stir a solution of the product of step A (0.50 g) and 2-methylthio-N-methylimidazole (0.40 g) at 0° to 5° C., in dichloromethane and add thereto diisopropylamine (0.2 g). After 5 minutes wash the solution with 10% aqueous tartaric acid, dry and evaporate and purify the residue by TLC to obtain allyl-(5R,6S,8R)-6-(1-hydroxyethyl)-2-(1-methyl-2-imidazolylmethylthio)-penem-3-carboxylate.

C. Treat the product from step B by the procedure described in Example 14, step B to obtain (5R,6S,8R)-2-(1-methyl-2-imidazolylmethylthio)-6-(1-hydroxyethyl)-penem-3-carboxylic acid.

EXAMPLE 16

(5R,6S,8R)-6-(1-Hydroxyethyl)-2-(1-methyl-2-imidazolylmethylthio)-penem-3-carboxylic acid

A. (3S,4R,5R)-3-(1-Trichloroethoxycarbonyloxyethyl)-4-[(1-methyl-2-imidazolylmethylthio)-carbonothioylthio]-azetidin-2-one

To a solution of ethanol (50 ml) containing 1-N sodium hydroxide (20 ml) and 2-methylthio N-methylimidazole (2.5 g) add carbon disulfide (4 ml) dropwise, stir ten minutes, then add dropwise to a solution of (3S,4R,5R)-4-[1-(2-methoxy-1,2-dioxoethyl)]-3-(1-trichloroethoxycarbonyloxyethyl)-4-chloroazetidin-2-one (4.1 g) in ethanol. Stir the reaction mixture until TLC analysis indicates no starting compound is present (about 4 hours) then dilute with ethyl acetate wash the solution with saturated sodium

chloride, dry the solution over magnesium sulfate, evaporate, and chromatograph the resulting residue on silica gel (40 g) eluting with 30% etherhexane. Combine the like elutes as determined by the TLC and evaporate to a residue which is the title compound. I.R. = 5.65 u
NMR : 5.5ppm (1H,d,J = 2 cps)
3.4ppm (1H,1,J = 8 and 2 cps)

B. Allyl-(5R,6S,8R)-6-(1-trichloroethoxycarbonyloxyethyl)-2-(1-methyl-2-imidazolylmethylthio)-penem-3-carboxylate

To a solution of (3S,4R,5R)-3-(trichloroethoxycarbonyloxyethyl)-4-[(1-methyl-2-imidazolylmethylthio)-carbonothioylthio]-azetidine-2-one (0.73 g) in methylene chloride (6 ml) cooled to 10° C., add, with stirring, calcium carbonate (0.6 g) followed by allyloxalyl chloride (0.263 g, 1.2 eq).
Add dropwise a solution of diisopropylethylamine (0.32 ml, 1.2 eq.) in methylene chloride (1 ml), during 5 minutes while maintaining the temperature at 10°-15° C. After TLC shows no starting compound (15 mins) at 15° C, transfer the mixture to a separatory funnel using ethanol-free chloroform. Wash twice with ice/water, filter to remove excess calcium carbonate, dry over anhydrous sodium sulfate, and transfer to a 100 ml 3-neck flask. Adjust the volume of the solution to approximately 50 ml with chloroform and heat at reflux temperature while adding a solution of triethylphosphite (0.6 ml, 2 eq.) in chloroform (20 ml) over a 3 hour period. Reflux the mixture for an additional 18 hours, evaporate and chromatograph on 14 g silica gel, eluting with 25% ether-hexane, and evaporate the combined like elutes to obtain a residue comprising the title compound. Purify by crystallization from ether-hexane to obtain the title compound in crystalline form.

C. Allyl-(5R,6S,8R)-6-(1-hydroxyethyl)-2-(1-methyl-2-imidazolylmethylthio)-2-penem-3-carboxylate

Dissolve about 1.6 g of the product prepared according to the process of step B, in 15 ml of tetrahydrofuran, 1.5 ml of water and 1.5 ml of acetic acid at 0°-5° C., with stirring. Add 2.0 g of zinc dust and stir until thin layer chromatography indicates only a trace of starting material. Filter the reaction mixture, wash solids with ethylacetate, combine the organic solvents and wash successively with 10% aqueous tartaric acid, water and with aqueous sodium bicarbonate solution. Dry the solvent phase over magnesium sulfate and concentrate to a residue. Crystallize the residue from ether- hexane.

D. (5R,6S,8R)-6-(1-hydroxyethyl)-2-(1-methyl-2-imidazolylmethylthio)-penem-3-carboxylic acid

Treat the product from step C by the procedure described in Example 14B to obtain the title compound.

## EXAMPLE 17

### (5R,6S,8R)-6-(1-Hydroxyethyl)-2-(2-glycylaminoethylthio)-penem-3-carboxylic acid

A. Allyl-(5R,6S,8R)-6-(1-hydroxyethyl)-2-(2-N-[allyloxycarbonylglycylamino]ethylthio)-penem-3-carboxylate

Stir a solution of the product of preparation E (0.50 g) and the product of preparation F (0.58 g) at 0°-5° C in dichloromethane and add thereto diisopropylethylamine (0.2 g). After 5 mins. wash the solution with 10% aqueous tartaric acid, dry and evaporate and purify the residue by preparative TLC (eluting with EtOAc; product Rf 0.4) to obtain the title compound as a pale yellow foam.
PMR ($CDCl_3$): 1.35 (d, J = 7 Hz, 3H), 3.16 (m, 2H), 3.4-4.0 (m, 6H), 4.24 (m, 1H), 4.60 (d, J = 7.5 Hz, 2H), 4.63 (m, 2H), 5.2-5.6 (m, 4H), 5.75 (d, J = 1.5 Hz, 1H), 5.7-6.2 (m, 3H) and 7.20 (br. t, J = 7 Hz).

B.

Stir a mixture of the product of preparation C (0.225 g), 2-ethylhexanoic acid (0.20 g) and triphenylphosphine (0.05 g) at 30°-35°C. in $CH_2Cl_2$ (20 ml) under nitrogen and add thereto tetrakis-(triphenylphosphine)palladium (0.03 g). After 1.5 hour, collect the precipitate by centrifugation after adding ether (15 ml), wash with 3 x 10 ml of 4:1 ether:$CH_2Cl_2$ and dry under nitrogen to give the title compound as a cream powder.
IR (nujol): 3400, 1770, 1650 and 1590 $cm^{-1}$.

EXAMPLE 18

A.

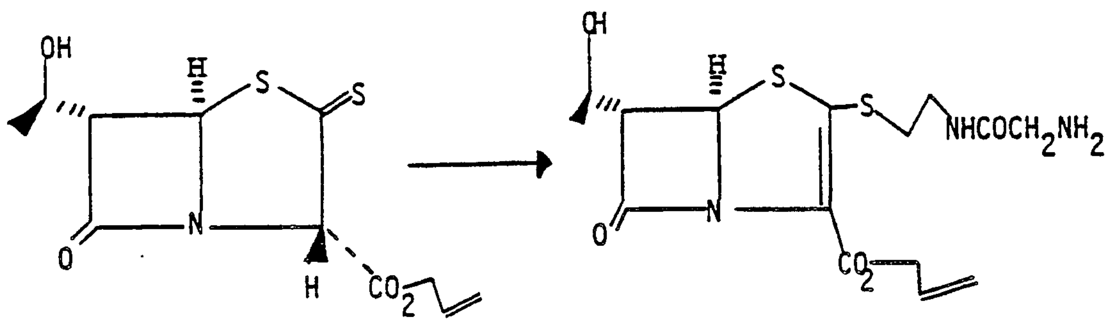

To a solution of 0.20 g of an equilibrium mixture of allyl (5R,6S,8R)-6-(1-hydroxyethyl)-2-thione-penam-3-carboxylate and allyl (5R,6S,8R)-6-(1-hydroxyethyl)-2-thiol-penem-3-carboxylate and diisopropylethylamine (0.15 g) in dry acetonitrile (4 ml) add 1-iodo, 2-(N-allyloxycarbonylglycylamino)-ethane (0.23 g). After 1 hour at room temperature dilute with aqueous tartaric acid and dry over magnesium sulfate. Evaporate and isolate the residue from dichloromethane to give allyl (5R,6S,8R)-6-(1-hydroxyethyl)-2-( 2[N-allyloxycarbonylglycylamino]ethylthio)-penem-3-carboxylate, as a pale yellow foam.

B. Treat the product from step A by following exactly the procedure described in Example 17, step B, to obtain (5R,6S,8R)-6-(1-hydroxyethyl)-2-(glycylaminoethylthio)-penem-3-carboxylic acid.

EXAMPLE 19

(5R,6S,8R)-6-(1-Hydroxyethyl)-2-[2-glycylamino)ethylthio]-penem-3-carboxylic acid

A. (3S,4R,5R)-3-(1-trichloroethoxycarbonyloxyethyl)-4-[(2-allyloxycarbonylglycylaminoethylthio)-carbonothioylthio]-azetidin-2-one

To a solution of ethanol (50 ml) containing 1-N sodium hydroxide (20 ml) and 2-(N-allyloxycarbonylglycylamino)-ethane thiol (5.0 g) add carbon disulfide (4 ml) dropwise. Stir for 10 minutes, then add dropwise to a solution of (3S,4R,5R)-[1-(2-methoxy-1,2-dioxoethyl)-3-(1-trichloroethoxycarbonyloxyethyl)-4-chloroazetidin-2-one (4.1 g) in ethanol. Stir the reaction mixture until TLC analysis indicates no starting material is present (about 4 hours) then dilute with ethyl acetate, wash the solution with saturated sodium chloride, dry the solution over magnesium sulfate, evaporate, and chromatograph the resulting residue on silica gel (40 g) eluting with 30% ether-hexane. Combine the like eluates as determined by the TLC and evaporate to a residue which is the title compound of this step.

B. Allyl-(5R,6S,8R)-6-(1-trichloroethoxycarbonyloxyethyl)-2-[(2-N-allyloxycarbonylglycylamino)ethylthio]-penem-3-carboxylic acid

To a solution of (3S,4R,5R)-3-(trichloroethoxycarbonyl-4-[2-(N-allyloxycarbonylglycylamino)-ethylthio]-carbonothioylthio]-azetidin-2-one (0.85 g) in methylene chloride (6 ml) cooled to about 10° C, add, with stirring, calcium carbonate (0.6 g) followed by allyloxalylchloride (0.263 g, 1.2 eq.) in methylene chloride (1 ml), over 5 minutes while maintaining the temperature in the range 10°-15° C. After TLC indicates starting compound is no longer present, transfer the mixture to a separating funnel using ethanol-free chloroform. Wash twice with ice/water, filter to remove excess calcium carbonate, dry over anhydrous sodium sulfate, and transfer to a 100 ml 3-neck flask. Adjust the volume of the solution to about 50 ml with chloroform and heat at reflux temperature while adding a solution of triethylphosphite (0.6 ml, 2 eq.) in chloroform (20 ml) over 3 hours. Reflux the mixture for an additional 18 hours, evaporate and chromatograph on 14 g silica gel, eluting with 25% ether-hexane, and evaporate the combined like eluates to obtain a residue comprising the title compound. Purify by crystallization from ether-hexane to obtain the title compound.

C. Allyl-(5R,6S,8R)-6-(1-hydroxyethyl)-2-[(2-N-allyloxyglycylamino)ethylthio]-penem-3-carboxylic acid

Dissolve out 1.8 g of the product obtained from step B, in 15 ml tetrahydrofuran, 1.5 ml of water and 1.5 ml acetic acid at 0°-5° C with stirring.

Add 2.0 g of zinc dust and stir until thin layer chromatography indicates only a trace of starting material. Filter the reaction mixture, wash solids with ethyl acetate, combine the organic solvents and wash successively with 10% aqueous tartaric acid, water and with aqueous sodium bicarbonate solution. Dry the solvent phase over magnesium sulfate and concentrate to a residue. Isolate the residue from ether-hexane to obtain the title compound.

D.

Treat 0.225 g of the compound obtained from step C by the procedure described in Example 17, step B, to obtain the title compound of this Example as a cream powder.

## EXAMPLE 20

Sodium-(5R,6S,8R)-6-(1-hydroxyethyl)-2-(2-methylcarbamoylethylthio)-penem-3-carboxylate

A. Allyl-(5R,6S,8R)-6-(1-hydroxyethyl)-2-(2-methylcarbamoylethylthio)-penem-3-carboxylate

Stir a solution of the penem allyl ester sulfoxide (2.55 g) prepared in Preparation E in $CH_2Cl_2$ (40 ml) with 3-mercapto-N-methylpropionamide (1.92 g) and cool to -10° C. Add diisopropylethylamine (0.6 ml) and continue stirring at -10° C. for 0.5 hour. Add ether (50 ml) and collect the precipitate and wash with ether. Stir the resulting solid with $CH_2Cl_2$ (20 ml) for 0.5 hour at 0° C., collect and dry to give the title compound as a white powder. m.p. 176°-178° C.

IR(nujol suspension): max 3400, 3300, 1775,1695, 1635, 1560 and 1510 $cm^{-1}$.

B. Sodium-(5R,6S,8R)-6-(1-hydroxyethyl)-2-(2-[methylcarbamoyl]-ethylthio)-penem-3-carboxylate

Stir a suspension/solution of the compound obtained in preparation A (0.66 g) in dry THF (50 ml) containing sodium 2-ethyl-hexanoate (0.305 g) and triphenylphosphine (0.12 g) at 25° C. under $N_2$ and add Pd $(PPh_3)_4$ (0.08 g). After 1.5 hour, add hexane (50 ml) and collect the crude product by centrifuge, wash with 2 x 20 ml ethyl acetate and partition with $H_2O$ (50 ml) - ethyl acetate (50 ml). Treat the aqueous phase with $N_2$ to remove dissolved organics then filter through 5 g of reverse phase C-18 silica gel, washing with

$H_2O$. Treat the filtrate with 5 g of DARCO active carbon, stir 0.5 hour filter, wash with $H_2O$ and lyophilize to give the title compound as a buff powder.
PMR ($D_2O$): 1.30 (d, J=7 Hz; 3H), 2.63 (m, 2H), 2.72 (s, 3H), 3,14 (m, 2H), 3.91 (dd, J=1.5 and 8 Hz, 1H), 4.25 (pentet, J=8 Hz, 1H) and 5.69 (d, J=1.5 Hz, 1H).

## EXAMPLE 21

### Sodium-(5R,6S,8R)-6-(1-hydroxyethyl)-2-(2-methylcarbamoylethylthio)-penem-3-carboxylate

A. (3S,4R,5R)-3-(1-trichloroethoxycarbonyloxyethyl)-4-[(2-methylcarbamoylethylthio)-carbonothioylthio]-azetidin-2-one

To a solution of ethanol (50 ml) containing 1-N sodium hydroxide (20 ml) and 3-mercapto-N-methylpropionamide (2.6 g) add carbon disulfide (4 ml) dropwise. Stir for 10 minutes, then add dropwise to a solution of (3S,4R,5R)-[1-(2-methoxy-1,2-dioxoethyl)-3-(1-trichloroethoxycarbonyloxyethyl)-4-chloroazetidin-2-one (4.1 g) in ethanol. Stir the reaction mixture until TLC analysis indicates no starting material is present (about 4 hours) then dilute with ethyl acetate, wash the solution with saturated sodium chloride, dry the solution over magnesium sulfate, evaporate the solvent and chromatograph the resulting residue on silica gel (40 g) eluting with 30% ether-hexane. Combine like fractions as determined by TLC and evaporate to obtain the title compound of this step.

B. Allyl-(5R,6S,8R)-6-(1-trichloroethoxycarbonyloxyethyl)-2-(2-methylcarbamoylethylthio)-penem-3-carboxylate

To a solution of the azetidinone obtained in step A (1.1 g) in methylene chloride (6 ml) cooled to about 10° C, add, with stirring, calcium carbonate (0.6 g) followed by allyloxalylchloride (0.263 g, 1.2 eq.) in methylene chloride (1 ml), over 5 minutes, while maintaining the temperature in the range 10° to 15° C. After TLC indicates starting compound is no longer present transfer the mixture to a separating funnel using ethanol-free chloroform. Wash twice with ice/water, filter to remove excess calcium carbonate, dry over anhydrous sodium sulfate, and transfer to a 100 ml 3-neck flask.

Adjust the volume of the solution to about 50 ml with chloroform and heat at reflux temperature while adding a solution of triethylphosphite (0.6 ml, 2 eq.) in chloroform (20 ml) over 3 hours. Reflux the mixture for an additional 18 hours, evaporate and chromatograph on 14 g silica gel, eluting with 25% ether-hexane and evaporate the combined like eluates to obtain a residue comprising the title compound. Purify by crystallisation from methylene chloride to give the title compound.

C. Allyl-(5R,6S,8R)-6-(1-hydroxyethyl)-2-(2-methylcarbamoylethylthio)-2-penem-3-carboxylate

Dissolve about 1.6 g of the product prepared according to the process of step B, in 15 ml of tetrahydrofuran, 1.5 ml of water and 1.5 ml of acetic acid at 0°-5° C. With stirring. Add 2.0 g of zinc dust and stir until thin layer chromatography indicates only a trace of starting material. Filter the reaction mixture, wash solids with ethyl acetate, combine the organic solvents and wash successively with 10% aqueous tartaric acid, water and with aqueous sodium bicarbonate solution. Dry the solvent phase over magnesium sulfate and concentrate to a residue. Crystallize the residue from ether-hexane.

D. Sodium-(5R,6S,8R)-6-(1-hydroxyethyl-2-(2-[methylcarbamoyl]-ethylthio)-penem-3-carboxylate.

Treat 0.66 g of the compound obtained in preparation C by the procedure described in Example 20, step B to obtain the title compound as a buff powder.

EXAMPLE 22

Allyl-(5R,6S,8R)-6-(1-hydroxyethyl)-2-[2-methylcarbamoyl)ethylthio]-penem-3-carboxylate

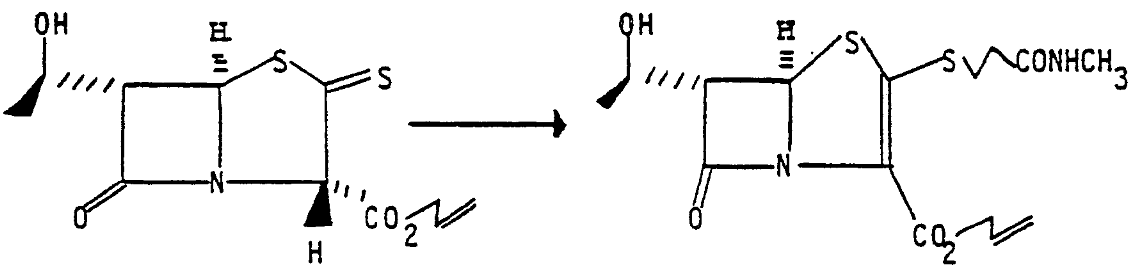

To a solution of 0.20 g of an equilibrium mixture of allyl (5R,6S,8R)-6-(1-hydroxyethyl)-2-thione-penem-3-carboxylate and allyl (5R,6S,8R)-6-(1-hydroxyethyl)-2-thiol-penem-3-carboxylate, and diisopropylethylamine (0.15 g) in dry acetonitrile (4 ml) add N-methyl-3-iodopropionamide (0.2 g). After 1 hour at 25°C., dilute with ethyl acetate (25 ml), wash with aqueous tartaric acid and dry ($MgSO_4$). Evaporate and crystallise the residue from dichloromethane to give the title compound.

The (5R,6R,8S) isomers corresponding to the (5R,6S,8R) compounds obtained in the above Examples can be obtained by following similar procedures to those described in the Examples but using appropriate stereospecific starting material namely (3R,4R,5S)-3-(1-trichlorocarboxyloxyethyl)-4-(triphenylmethylthio)-azetidin-2-one.

The (5R,6S,8R)-allyl-2-substituted thio-6-(1-hydroxyethyl)-penem-3-carboxylates may readily be converted to their corresponding alkali metal salts by the procedure described in our European Patent Application Publication No. 0013663. In this procedure the allyl group can be removed by utilizing a suitable aprotic solvent such as tetrahydrofuran, diethyl ether or methylene chloride, with potassium or sodium 2-ethylhexanoate and a mixture of a palladium compound and triphenyl phosphine as the catalyst to afford the corresponding penem sodium or potassium salt directly.

By following the experimental procedures set forth above using appropriate starting materials, the following compounds can be prepared.

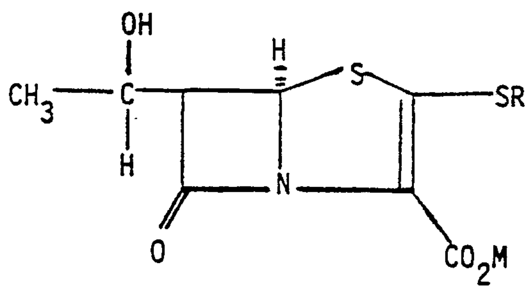

M = hydrogen or an alkali metal (preferably sodium or potassium) salt.

| Example No. | R | |
|---|---|---|
| 23 | $-CH_2CH_3$ | Data for the potassium salt (5R,6S,8R) isomer<br>NMR $\delta$ = 1.25-1.49, 6H;<br>$\delta$= 2.76-3.14, 2H;<br>$\delta$= 3.85-3.94, 1H;<br>$\delta$= 4.12-4.37, 1H;<br>$\delta$= 5.65-5.67, 1H, d; ($D_2O$)<br>Rotation:<br>$[\alpha]^{26}_{D}$ = -145.2°<br>IR:<br>1600 $cm^{-1}$ and 1770 $cm^{-1}$ (nujol). |
| 24 | $-CH_2CH_2CH_3$ | Data for the sodium salt (5R,6S,8R) isomer.<br>A brown powder.<br>IR spectrum (nujol) $\nu$ max. 1770 and 1600 $cm^{-1}$. |
| 25 | $-CH_2CH_2F$ | Data for the sodium salt (5R,6S,8R) isomer.<br>$[\alpha]^{26}_{D}$ = +150.8° [$H_2O$]<br>NMR, $D_2O$ 5.75, 1H, d (J=1 Hz); 5.05 and 4.4 (2 triplets $-CH_2F$) 2H, (J=1.8 Hz, 9 Hz); 4.3, 1H, m; 3.95, 1H, dd, J=1 and 8Hz; 3.3, 2H m; 2.2, 1H |

| | | |
|---|---|---|
| | | (EXCHANGEABLE - OH); 1.3, 3H, d, J=9 Hz. |
| 26 | $-CH_2CH_2OH$ | Data for the sodium salt (5R,6R,8S) isomer. A pale brown powder 'H NMR ($D_2O$) : 1.24 (d, 3,J= 7), 3.0 (m,2), 3.77 (t,2,J= 7) 3.63 (dd,1, J= 6.2), 4.15 (m,1) 5.60 (d,1,J= 2). |
| 27 | $-CH_2CH_2NH_2$ | free acid (5R,6S,8R) isomer, is a cream powder. |
| 28 | $-CH_2CH_2OCH_3$ | |
| 29 | - phenyl | |
| 30 | $-CH_2CH_2SCH_3$ | |
| 31 | $-CH_2C_6H_5$ | potassium salt (5R,6S, 8R) isomer is a light brown solid. |

| | | |
|---|---|---|
| 32 | $-CH_2CH_2CH_2F$ | |
| 33 | $-CH_2CH_2Cl$ | Data for the sodium salt (5R,6S,8R) isomer is as follows: $D_2O$; $\delta$ 5.6 (1H,d), 4.2 (1H, t), 3.75 (3H, m), 3.2 (2H,m), 1.2 (3H,d). |
| 34 | $-CH_2OCH_3$ | |
| 35 | $-CH_2-S-CH_3$ | |
| 36 | $-CH_2-S-CH_2-C_6H_5$ | |
| 37 | $CH_2CH_2-S-C_6H_5$ | Sodium salt (5R,6S,8R) isomer is a yellow powder. $D_2O$; $\delta$ 7.3(5H, m), 5.5 (1H, d), 4.1 (1H, t), 3.75 (1H, d of d), 3.1 (4H, m), 1.25 (3H, d) |

| | | |
|---|---|---|
| 38 | 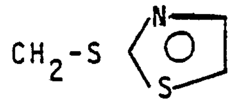 | |
| 39 | 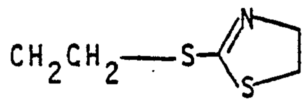 | Sodium salt (5R,6S,8R) isomer is off-white solid. $D_2O$: δ 5.58 (1H, s), 5.0 (1H, m), 3.8-4.5 (5H, m), 3.3 (4H, m), 1.2 (3H,d). |
| 40 | $-CH_2CN$ | Data for the sodium salt (5R,6S,8R) isomer is as follows:<br>90mHz NMR $D_2O/CD_3CN$<br>120ppm, D J=6Hz, 3H<br>5.70ppm, D J=1Hz, 1H. |
| 41 | $-CH_2\overset{O}{\overset{\|}{C}}-CH_3$ | Data for the sodium salc (5R,6S,8R) isomer is as follows:<br>90mHz NMR $D_2O$ 1.30ppm, D J=6Hz, 3H<br>2.40ppm, s, 3H<br>3.90ppm, DD 6Hz, 1Hz, 1H,<br>4.25ppm, m, 1H,<br>5.65ppm D J=1Hz, 1H<br>Mass spectrum<br>Parent Ion 324 mass units<br>100% Ion 117 mass units |

| | | |
|---|---|---|
| 42 | $CH_2CO_2CH_3$ | Potassium salt (5R,6S,8R) is a pale brown solid |
| 43 | $CH_2CH_2CO_2CH_3$ | |
| 44 | $CH_2\overset{O}{\overset{\Vert}{C}}NH_2$ | |
| 45 | $CH_2\overset{O}{\overset{\Vert}{C}}NHCH_3$ | |
| 46 | $CH_2CH_2N(CH_3)_2$ | |

| | | |
|---|---|---|
| 47 | $-CH_2CH_2CN$ | Data for the sodium salt is as follows:<br>60mH NMR $D_2O$<br>1.30ppm, D J=6Hz, 3H<br>2.80-3.40ppm, m, 4H,<br>3.90ppm, dd J=6,Hz, 1H,<br>4.20ppm, m, 1H,<br>5.70ppm, D J=1Hz, 1H |
| 48 | 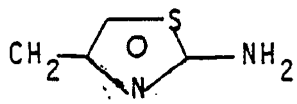 | Data for the free acid (5R,6S,8R) isomer is as follows:90 mHz NMR ($D_2O$)<br>$\delta$ = 6.52, 1H, S; 5.52 1H, d (J=1.5Hz);<br>4.28-4.04, 1H, m; 4.0-3.87, 2H, m; 1.19, 3H, d (J=6Hz). |
| 49 | 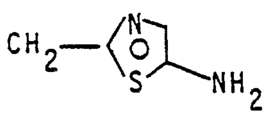 | |

| | | |
|---|---|---|
| 50 | $CH_2COONa$ | Data for the double sodium salt (M=Na) (5R,6S,8R) isomer is as follows:<br><br>90 mHz NMR ($D_2O$)<br>$\delta$ = 6.64, 1H, d(J=1.5Hz); 4.3-4.06, 1H, m; 3.87, 1H, dd (J=1.5, 6Hz); 3.62, 2H, s; 1.25, 3H, d(J=6Hz). |
| 51 | $CH_2CH_2COONa$ | Data for the disodium salt (M=Na) (5R,6S,8R) isomer.<br>$[\alpha]^{26}_{D}$ ($H_2O$) = +153.7°.<br><br>PMR($D_2O$) : 1.36 (d, J=Hz, 2H), 2.6(m,2H), 3.2(m,2H), 3.93(dd,J=8 and $2H_z$, 1H), 4.28(m,1H) and 5.71 (d, J=$2H_z$, 1H). |

| | | |
|---|---|---|
| 52 | 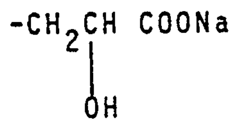 | Data for the double sodium salt (5R,6S,8R) isomer is as follows:<br><br>60 mHz NMR $D_2O$<br>1.15ppm, d J=6Hz, 3H<br>2.8-3.40ppm, m, 2H<br>3.85ppm, m, 1H<br>4.20ppm, m, 2H<br>5.60ppm, D J= 1Hz, 1H. |
| 53 | 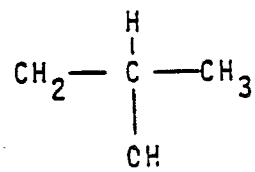 | Sodium salt (5R,6S,8R) isomer, is a tan solid. |
| 54 | 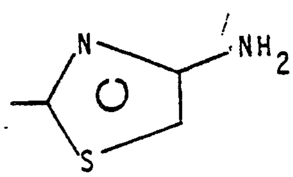 | |
| 55 | 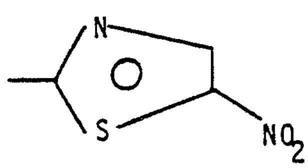 | |

56

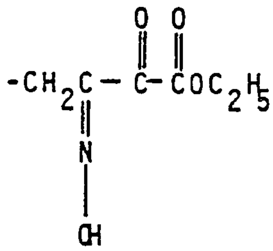

57

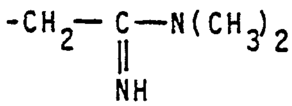

Data for the sodium salt (5R,6S,8R) isomer is as follows:

90 mHz NMR $D_2O$

1.25ppm, D J=6Hz, 3H

3.17ppm, s, 3H

3.30ppm, s, 3H

3.8-4.3ppm, m, 2H

5.70ppm, D J=1Hz, H

58

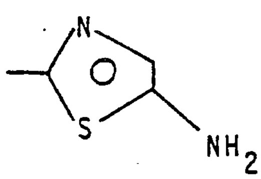

59 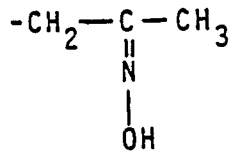

Data for the sodium salt is as follows:

60 mHz NMR $D_2O$

1.20ppm, D J=6Hz, 3H
1.95ppm, s, 3H
3.60ppm, s, 2H
3.80ppm, DD J=6Hz, 1Hz, 1H,
4.20ppm, m, 1H
5.60ppm, D J=1Hz, 1H.

60 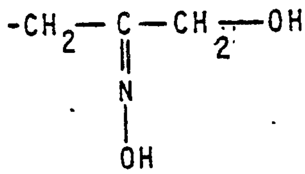

61 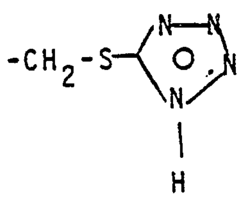

62 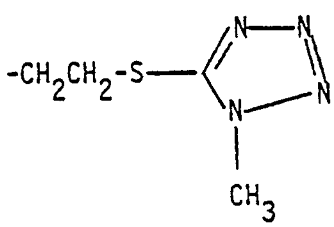

Sodium salt is a yellow solid.
$D_2O$: $\delta$ 5.4 (1H,d); 4.1(1H, t); 3.8 (3H,s); 3.73 (1H, d of d); 2.9-3.6 (4H, m), 1.13 (3H, d).

63 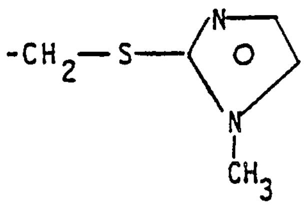

64 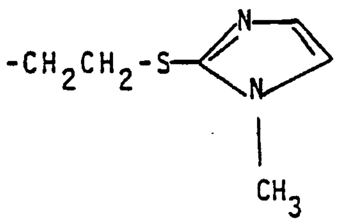

Sodium salt is a brown solid. $D_2O$: $\delta$ 7.1 (2H,d); 5.5 (1H, d), 4.1(1H,t); 3.8 (3H, s, 1H, d); 3.1 (4H, m); 1.3 (3H, d).

65 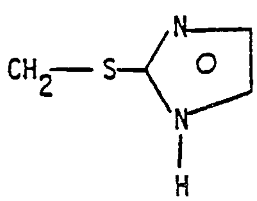

66 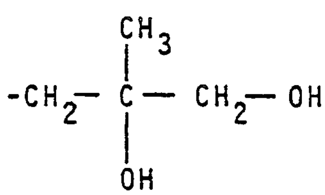

67 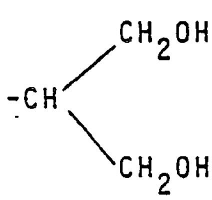

68 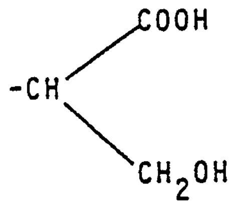

69 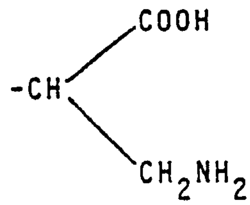

70 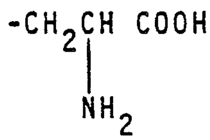

71 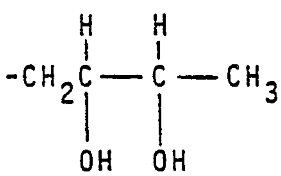

72 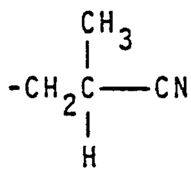

73 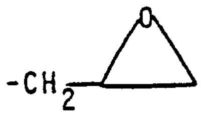

74 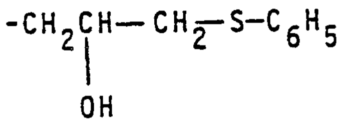

75 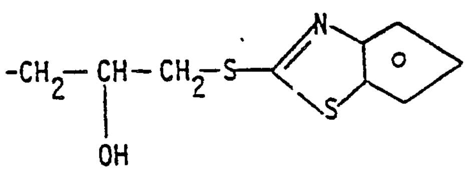

76 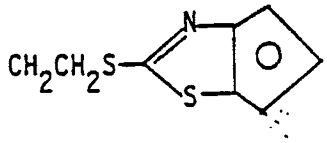

Data for sodium salt (5R,6S,8R) isomer is as follows:

$D_2O$: δ 7.8-7.0 (4H,m), 5.25 (1H, d), 4.0 (1H,t), 3.5 (1H, d), 2.7-3.4 (4H, m) 1.3 (3H,d).

77 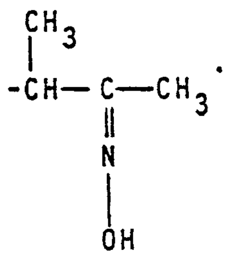

78 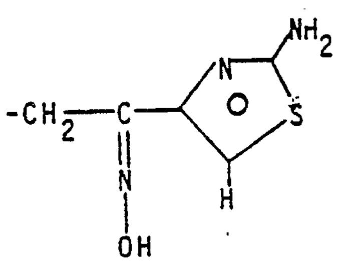

79 $-CH_2-CH=N-OH$

80 $-CH_2-CH=N-O-CH_2-COOH$

81 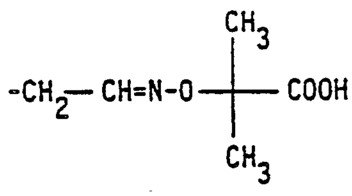

82 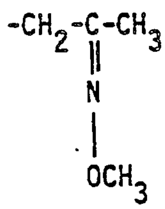

83 $-CH_2-CH=N-OCH_3$

84 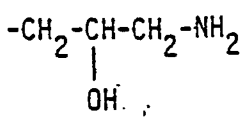

85 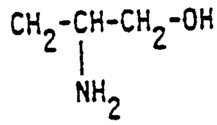

86 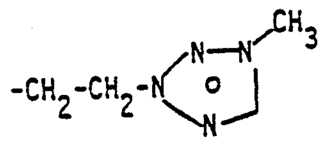

87 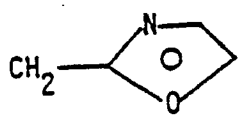

88 A 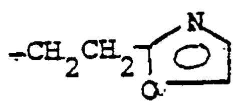

88 B 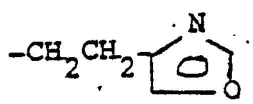

88 C 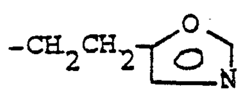

89 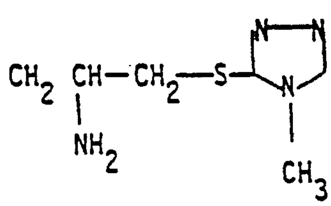

90

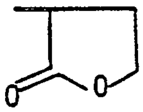

91

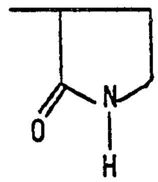

92

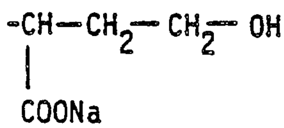

93

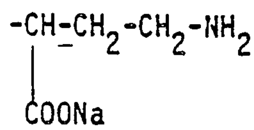

94 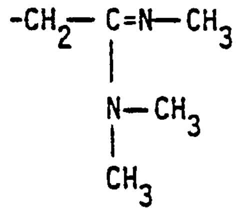

95 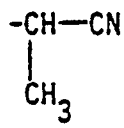

96 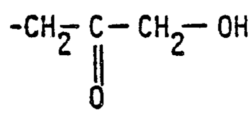

97 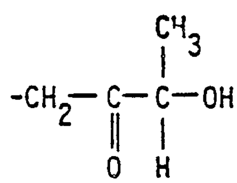

98 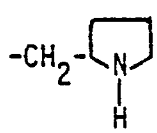

99 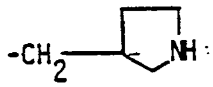

100 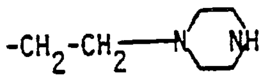

101 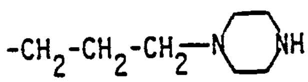

102 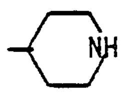

Data for the sodium salt (5R,6S,8R) isomer is as follows:

90 mHz NMR $D_2O$

1.20ppm, D J=6Hz, 3H
1.5-2.3ppm, m, 4H
2.7-3.5ppm, m, 4H
3.75ppm, DD 6Hz, 1Hz, 1H
4.15ppm, m, 1H
5.60ppm D J=1Hz, 1H

Mass spectrum
Parent ion at 348 (330 + 18 $NH_4^+$ )
100% ion at 152

103

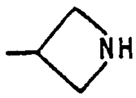

104

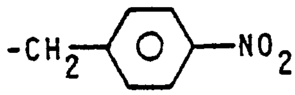

105

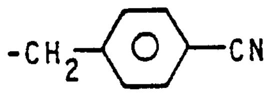

106

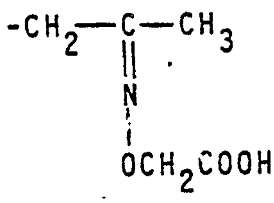

107

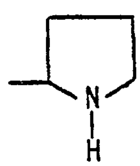

108 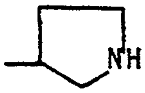

109 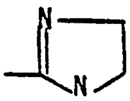

Sodium salt (5R,6S,8R) isomer is a light brown solid.

$D_2O$; d 7.2 (1H,s), 6.95 (1H, s), 5.2 (1H, d), 3.95 (1H, m), 3.6 (3H, 1H, d of d), 1.05 (3H, d).

110 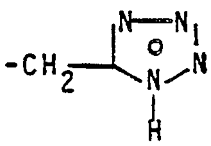

111 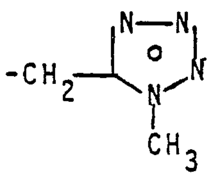

112 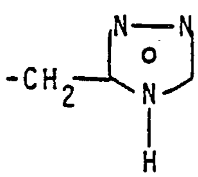

114 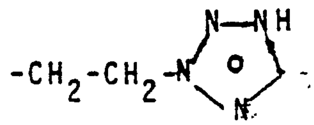

115 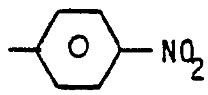

116 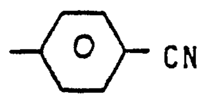

117 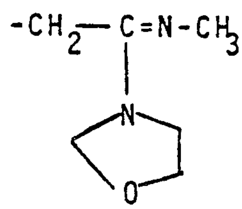

118 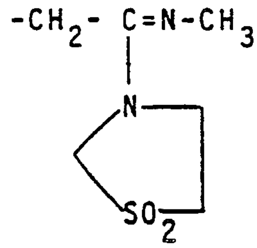

119 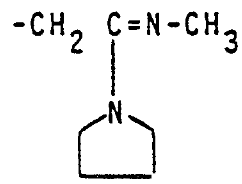

120 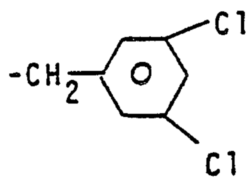

121 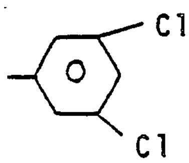

122 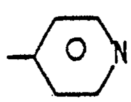

123 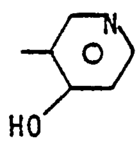

124 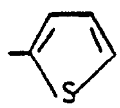

125 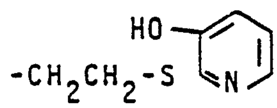

Data for sodium salt, (5R,6S,8R) isomer, is a brown powder.

$D_2O$: δ7.75 (1H,m); 7.0 (2H,d); 5.52 (1H,d); 4.1 (1H,m); 3.78 (1H,d); 3.1 (4H,br); 1.17 (3H,d).

126 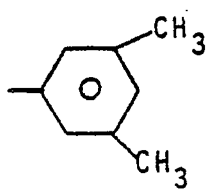

127 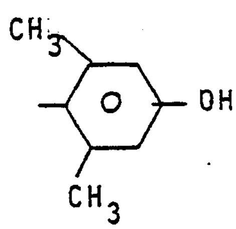

128 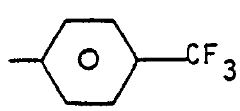

129 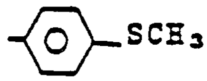

130 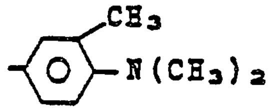

131 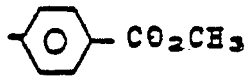

132 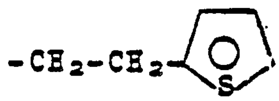

133 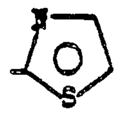

134 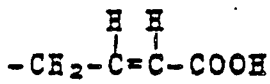

135 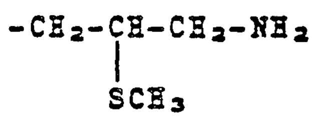

136 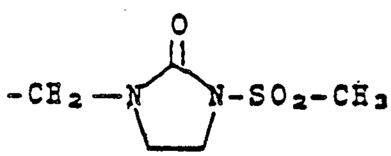

137 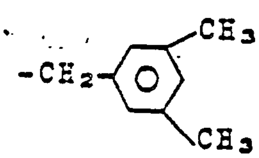

138 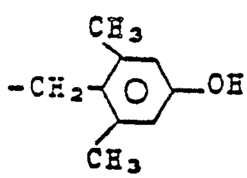

139 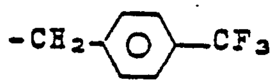

140 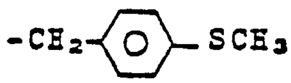

141 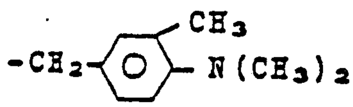

142 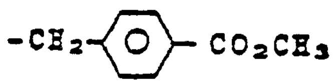

143 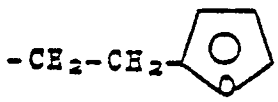

144 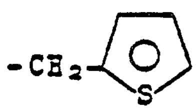

145 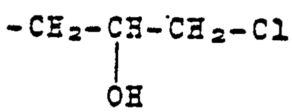

146 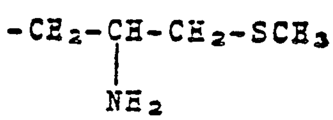

147 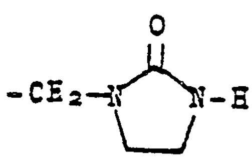

148 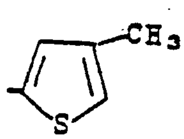

149 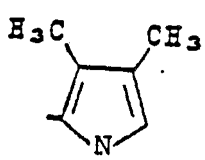

150 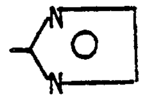

151 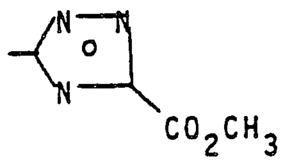

152 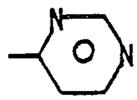

153 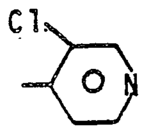

154 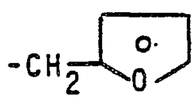

155 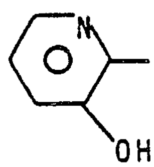

156 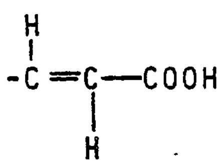

A study of in vitro activity of several representative novel penems of this invention, against gram +ve and gram -ve bacteria, is given in the Table below. The study was carried out by microlitre using Müller Hinton Agar. The number of organisms of each group is indicated in brackets in the left-hand column. The figures given are the geometric mean MICs based on, the various values obtained for the individual strains of each group.

Compound

1. (5R,6S,8R)-6-(1-hydroxyethyl)-2-[2-(aminoacetylamino)-ethylthio]-penem-3-carboxylic acid.

2. (5R,6S,8R)-6-(1-hydroxyethyl)-2-[2-(N-methylcarbamoyl)-ethylthio]-penem-3-carboxylic acid.
3. (5R,6S,8R)-6-(1-hydroxyethyl)-2-(2,3-dihydroxypropylthio)-penem-3-carboxylic acid.
4. (5R,6S,8R)-6-(1-hydroxyethyl)-2-(1-methyl-2-imidazolylmethylthio)-penem-3-carboxylic acid.
5. (5R,6S,8R)-6-(1-hydroxyethyl)-2-(N,N-dimethylcarbamimidoylmethylthio]-penem-3-carboxylic acid.

GEOMETRIC MEAN MICs mcg/ml    24 Hours

| COMPOUND | 1 | 2 | 3 | 4 | 5 |
|---|---|---|---|---|---|
| Gram +ve<br>B Subtilis (1)<br>Sarcina lutea (1)<br>Staphylococcus (18)<br>Streptomyces (2)<br>total 22 strains | 0.47 | 0.41 | 0.58 | 0.33 | 0.35 |
| Gram -ve<br>E. Coli (11)<br>Enterobacter (3)<br>Klebsiella (10)<br>Morganella/<br>Providencici (6)<br>Salmonella (3)<br>Serratia (4)<br>total 37 strains | 2.1 | 1.0 | 1.2 | 1.2 | 2.0 |

The compounds 1 to 5 listed above, whether as free acid, salt or metabolisable ester exhibit low protein binding, good stability in human serum, and their metabolites have little or no unpleasant odour.

## FORMULATIONS

In the following Examples the term "drug" indicates any one of the pharmaceutically active compounds of formula 1, especially
(5R,6S,8R)-6-(1-hydroxyethyl)-2-(2-glycylaminoethylthio)-penem-3-carboxylic acid,
(5R,6S,8R)-6-(1-hydroxyethyl)-2-(1-methyl-2-imidazolylmethylthio)-penem-3-carboxylic acid,
(5R,6S,8R)-2-(2-amino-4-thiazolylmethylthio)-6-(1-hydroxyethyl)-penem-3-carboxylic acid,
(5R,6S,8R)-2-(N,N-dimethylcarbimidoylmethylthio)-6-(1-hydroxyethyl)-penem-3-carboxylic acid,
(5R,6S,8R)-2-(2,3-dihydroxy-1-propylthio)-6-(1-hydroxyethyl)-penem-3-carboxylic acid,
(5R,6S,8R)-6-(1-hydroxyethyl)-2-(2-fluoroethylthio)-penem-3-carboxylic acid,
(5R,6S,8R)-2-(N-methylcarbamoylethylthio)-6-(1-hydroxyethyl)-penem-3-carboxylic acid,
or a pharmaceutically-effective equivalent amount of any of their pharmaceutically acceptable salts, especially sodium or potassium salts or metabolisable esters, or of any of the other compounds of formula I.

| Capsules | | | |
|---|---|---|---|
| No. | Ingredient | mg/capsule | mg/capsule |
| 1. | Active ingredient | 250 | 500 |
| 2. | Lactose USP | 100 | 50 |
| 3. | Corn Starch, Food Grade | 50 | 43.5 |
| 4. | Microcrystalline Cellulose NF | 95 | 50 |
| 5. | Magnesium Stearate NF | 5 | 6.5 |
| | Total | 500 | 650 |

Method of Manufacture

Mix Items nos. 1, 2, 3 and 4 in a suitable mixer for 10 -15 minutes. Add Item No. 5 and mix for 1 - 3 minutes. Fill the mixture into suitable two-piece hard gelatin capusles using an encapsulating machine.

| Tablet Formulation | | | |
|---|---|---|---|
| Item No. | Ingredient | mg/tablet | mg/tablet |
| 1. | Drug | 250 | 500 |
| 2. | Lactose, USP | 106 | 112 |
| 3. | Corn Starch, Food Guide as 10% paste in water | 20 | 40 |
| 4. | Corn Starch, Food Guide | 20 | 40 |
| 5. | Magnesium Stearate | 4 | 8 |
| | | 400 mg | 800 mg |

Method of Manufacture

Mix Items Nos. 1 and 2 in a suitable mixer for 10 - 15 minutes. Granulate the mixture with item No. 3. Pass the wet granulation through a coarse screen (1/4"). Dry the wet granules for 8 - 12 hours at 40° - 50° C. Using a suitable mill, pass the dried granules through a medium screen (No. 12 to No. 16). Add Item

No. 4 and mix for 10 - 15 minutes. Add Item No. 5 and mix further for 1-3 minutes. Compress the mixture to appropriate size and weight on a suitable tablet machine.

| Injectable Suspension Formulation | mg/ml |
|---|---|
| Sterile drug | 250.0 |
| Benzyl Alcohol | 9.0 |
| Methylparaben | 1.8 |
| Propylparaben | 0.2 |
| Sodium Carboxymethylcellulose | 5.0 |
| Polyethylene Glycol 4000 | 10.0 |
| Povidone | 5.0 |
| Sodium Citrate | 15.0 |
| Disodium Edetate | 0.1 |
| Water for injection | q.s. |
| To make | 1.0 ml |

Method of Manufacture

Dissolve parabens in a portion of the water for injection by heating it to 65°-70°C. Cool to 25°-35°C. Charge and dissolve benzyl alcohol, sodium citrate, disodium edetate, PEG 4000, povidone and sodium carboxymethylcellulose. Filter the solution and sterilize by autoclaving. Make a slurry of the sterile active and pass it through a colloid mill. Mix it well with solution from Step 3 and pass it through the mill. Bring the suspension to the final volume/weight and fill into sterile containers.

## Claims

1. A compound of the general formula

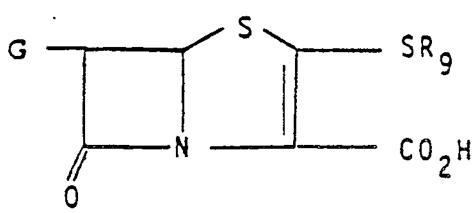

XII

in which G is 1-hydroxyethyl, $R_9$ is chosen from the group

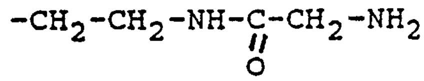

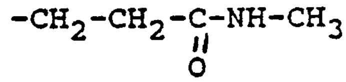

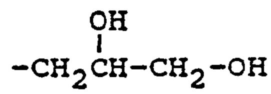

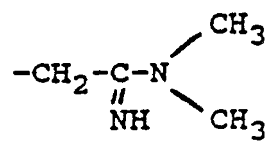

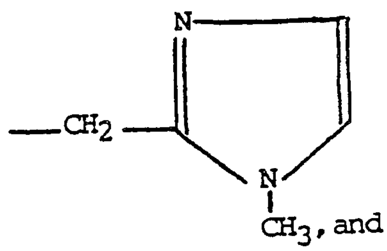

, and

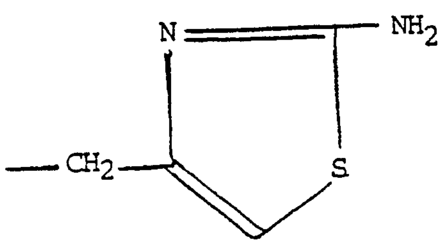

2. A compound according to claim 1 having the stereochemistry (5R,6S,8R) or (5R,6R,8S).

3. A pharmaceutical composition which comprises a compound as defined in claim 1 or 2 in combination with a pharmaceutically acceptable carrier or excipient.

Claims for the following Contracting State: AT

1. A process for producing a compound of the general formula

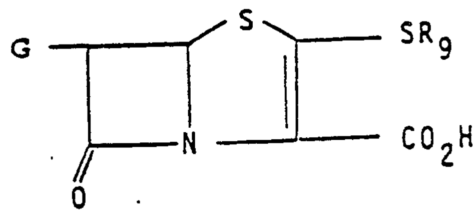

XII

in which G is 1-hydroxyethyl, $R_9$ is chosen from the group

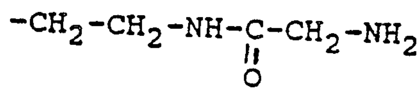

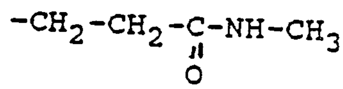

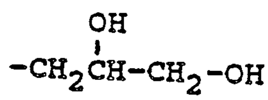

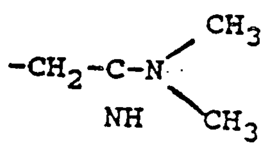

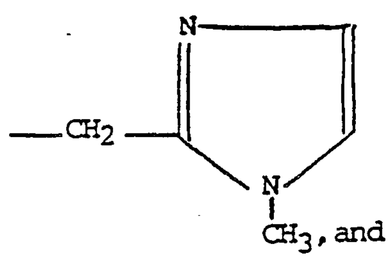

, and

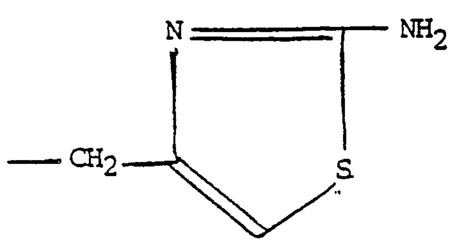

characterized in that either

(a) a tautomeric compound of formulas Va and Vb

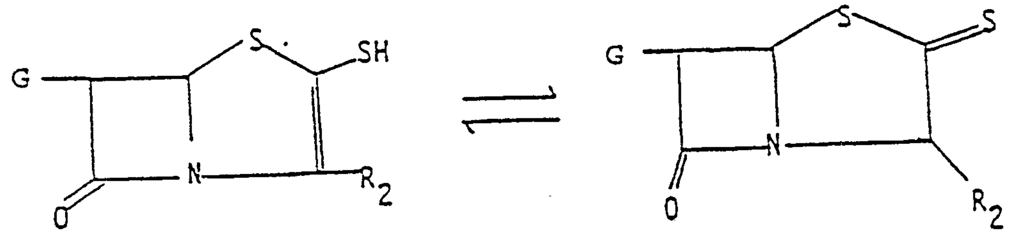

( Va )  ( Vb )

wherein G is as defined previously and $R_2$ is a protected carboxy group is subjected to an alkylation reaction;

(b) a compound of formula VII

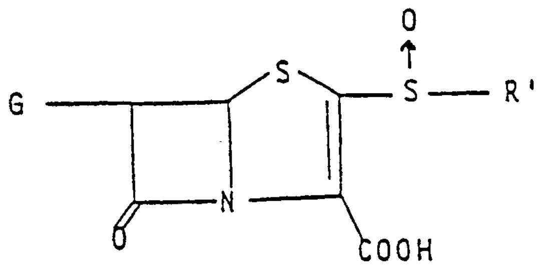

in which R' is an organic group different from the desired $R_9$ group is reacted with a thiol of formula VIII' or reactive derivative thereof

$R_9$-SH VIII'

in which $R_9$ is as defined previously; or

(c) a compound of formula IX

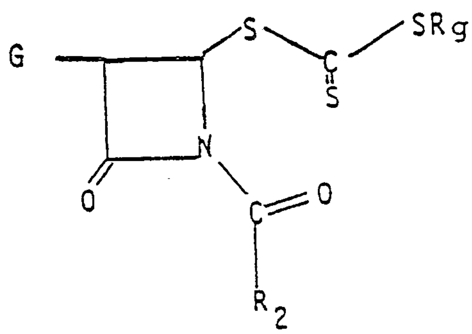

wherein G, $R_2$, and $R_9$ are as defined previously, is reacted with a trivalent organic phosphorous compound, wherein in the processes (a) to (c) above, any functional groups in the reactants may be protected by protecting groups, which are removed at any appropriate stage of the process, and a so-obtained compound of formula XII, if desired, is subjected to deprotection of a protected carboxyl group $R_2$ (if present) and is isolated as the free acid, a pharmaceutically acceptable salt or pharmaceutically acceptable ester.

2. The process of claim 1 wherein the compound produced has the stereochemistry (5R,6S,8R) or (5R,6R,8S).

3. A pharmaceutical composition comprising a compound of formula XII as defined in claims 1 or 2 in combination with a pharmaceutically acceptable carrier or excipient.

European Patent Office

# EUROPEAN SEARCH REPORT

Application Number

EP 89 11 3483

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 477 547 (SANKYO)<br>* Page 24, no. 83; pages 80-81, example 19; pages 89-90, example 32; claims *<br>--- | 1-3 | C 07 D 499/00<br>A 61 K 31/43 |
| Y | EP-A-0 002 210 (MERCK)<br>* Claims *<br>--- | 1-3 | |
| Y | EP-A-0 013 662 (SCHERING)<br>* Claims *<br>--- | 1-3 | |
| P,X | EP-A-0 087 792 (MERCK)<br>* Page 86, example 22; pages 88-91, compound 27; claims *<br>----- | 1-3 | |

TECHNICAL FIELDS SEARCHED (Int. Cl.4)

C 07 D 499/00
A 61 K 31/00

The present search report has been drawn up for all claims

| Place of search | Date of completion of the search | Examiner |
|---|---|---|
| THE HAGUE | 31-08-1989 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document